# EUROPEAN PATENT SPECIFICATION

(11) **EP 2 338 504 B1**
(45) Date of publication and mention of the grant of the patent: **06.05.2015**
(21) Application number: 10196012.8
(22) Date of filing: 10.03.2004
(51) Int. Cl.: A61K 38/16, A61K 38/17, C12N 15/866, C12N 15/63, C12N 15/64, C12N 5/10, C07K 14/435, C12Q 1/68, C12Q 1/70, A61P 39/00, A61K 38/00, A61K 47/48, B82Y 5/00, C07K 14/47, C12N 15/88

(54) **Vault and vault-like carrier molecules**
Gewölbe- und gewölbeähnliche Trägermoleküle
Voute et molécules porteueses de type voute

(30) Priority: 10.03.2003 US 453800 P
(43) Date of publication of application: 29.06.2011
(62) Divisional of application: 04719308.1
(73) Proprietor: The Regents of the University of California, Oakland, CA 94607 (US)
(72) Inventor: Rome, Leonard H., Tarzana, CA 91356 (US); Kickhoefer, Valerie A., Sherman Oaks, CA 91403 (US); Raval-Fernandes, Sujna, West Hills, CA 91307 (US); Stewart, Phoebe L., Brentwood, TN 37027 (US)
(74) Representative: Goodfellow, Hugh Robin

(56) References cited:
- STEPHEN A G ET AL: "Assembly of Vault-like Particles in Insect Cells Expressing only the Major Vault Protein", JOURNAL OF BIOLOGICAL CHEMISTRY 20010629 AMERICAN SOCIETY FOR BIOCHEMISTRY AND MOLECULAR BIOLOGY INC. US, vol. 276, no. 26, 29 June 2001 (2001-06-29), pages 23217-23220, XP002636434, DOI: DOI:10.1074/JBC.C100226200
- KICKHOEFER ET AL: "Vaults are the answer, what is the question?", TRENDS IN CELL BIOLOGY, ELSEVIER SCIENCE LTD, XX, vol. 6, no. 5, 1 May 1996 (1996-05-01), pages 174-178, XP022215182, ISSN: 0962-8924, DOI: DOI:10.1016/0962-8924(96)10014-3
- KONG L.B. ET AL: "Structure of the vault, a ubiquitous cellular component", STRUCTURE, 7/4 (371-379), 37 REFERENCE(S) CODEN: STRUE6 ISSN: 0969-2126, 15 April 1999 (1999-04-15), XP009094182,
- SUPRENANT K A: "Vault ribonucleoprotein particles: sarcophagi, gondolas, or safety dep", BIOCHEMISTRY, AMERICAN CHEMICAL SOCIETY, US, vol. 41, no. 49, 23 October 2002 (2002-10-23), pages 14447-14454, XP002985240, ISSN: 0006-2960, DOI: DOI:10.1021/BI026747E
- VAN ZON ET AL: "Structural domains of vault proteins: a role for the coiled domain in vault assembly", BIOCHEMICAL AND BIOPHYSICAL RESEARCH COMMUNICATIONS, ACADEMIC PRESS INC. ORLANDO, FL, US, vol. 291, no. 3, 1 January 2002 (2002-01-01), pages 535-541, XP002985241, ISSN: 0006-291X, DOI: DOI:10.1006/BBRC.2002.6472

## Description

### BACKGROUND

Vaults are ubiquitous, highly conserved cellular components found in phylogeny as diverse as mammals, avians, amphibians, the slime mold *Dictyostelium discoideum,* and the protozoan *Trypanosoma brucei.* Scanning transmission electron microscopic analysis has shown that the molecular mass of vaults is about 12.9 ± 1 MDa, and cryoelectronmicrograph single particle reconstruction has determined that vaults have an overall dimension of about 420 x 420 x 750 Å. Thus, vaults have a greater mass and size than many icosahedral viruses. The function of vaults is currently unknown.

Vaults are ribonucleoprotein particles comprising three different proteins, designated MVP, VPARP and TEP1, and between one and three different untranslated RNA molecules, designated vRNAs. For example, the rat *Rattus norvegicus* has only one form of vRNA per vault, while humans have three forms of vRNA per vault. The major vault protein, MVP, a 95.8 kDa protein in *Rattus norvegicus* and a 99.3 kDa protein in humans, is present in 96 copies per vault and accounts for about 75 % of the total protein mass of the vault particle. The two other proteins, the vault poly-ADP ribose polymerase, VPARP, a 193.3 kDa protein in humans, and the telomerase/vault associated protein 1, TEP1, a 292 kDa protein in *Rattus norvegicus* and a 290 kDa protein in humans, are each present in between about 2 and 16 copies per vault.

VPARP, is a poly ADP-ribosyl polymerase apparently unique to vaults. It includes a region of about 350 amino acids that shares 28 % identity with the catalytic domain of poly ADP-ribosyl polymerase, PARP, a nuclear protein that catalyzes the formation of ADP-ribose polymers in response to DNA damage. VPARP catalyzes an NAD-dependent poly ADP-ribosylation reaction, and purified vaults have poly ADP-ribosylation activity that targets MVP, as well as VPARP itself.

Cryo-electron microscopy studies have determined that the vaults are hollow, barrel-like structures with two protruding end caps and an invaginated waist. Regular small openings surround the vault cap. These openings are large enough to allow small molecules and ions to enter the interior of the vault. The volume of the internal cavity of the vault is about 5x10⁷ Å³, large enough to enclose two ribosomes.

Stephen A G et al. (2001, JBC. 276(26), 23217-23220) discloses that although the precise function of vaults is unknown, their wide distribution and highly conserved morphology in eukaryotes suggests that their function is essential and that their structure must be important for their function. Also disclosed is that vaults have been implicated in the phenomenon of multidrug resistance and as prognostic markers for chemotherapy failure. Stephen A G *et al.* (2001) concludes that understanding vault assembly will enable us to design agents that disrupt vault formation.

### SUMMARY

According to the present invention, there is provided a method of using vault-like particles as carrier molecules to an environmental medium. The method comprises a) providing vault-like particles comprising the one or more than one substance, wherein the vault-like particles: (i) comprise, consist essentially of or consist of a modified MVP; (ii) comprise a modified VPARP or modified portion of VPARP comprising at least 150 consecutive residues of VPARP; or (iii) comprise both a modified MVP and a modified VPARP or modified portion of VPARP comprising at least 150 consecutive residues of VPARP; and b) administering the vault-like particles to the environmental medium, wherein the one or more than one substance is selected from the group consisting of an enzyme, a pharmaceutical agent, a plasmid, a polynucleotide, a polypeptide, a chemical sensor, a fluorescent sensor, an ionic sensor, a microorganism sensor, an optical sensor or a pH sensor, or a combination thereof. In one embodiment, the vault-like particles are generated using recombinant technology.

In some embodiments, the vault-like particle further comprises TEP1 or modified TEP1.

According to another embodiment of the present invention, the vault-like particle comprises modified MVP. In one embodiment, the modified MVP comprises an amino acid sequence added to the N-terminal of the MVP which results in one or more than one substance-binding domain within the vault-like particle. In another embodiment, the one or more than one substance-binding domain is between 1 and 95 substance-binding domains. In another embodiment, the one or more than one substance-binding domain is 96 substance-binding domains. In another embodiment, the one or more than one substance-binding domain is greater than 96 substance-binding domains. In one embodiment, the one or more than one substance-binding domain within the vault-like particle is one or more than one heavy metal binding domain. In a preferred embodiment, the one or more than one heavy metal binding domain binds a heavy metal selected from the group consisting of cadmium, copper, gold and mercury. In a preferred embodiment, the peptide added to the N-terminal is a cysteine-rich peptide. In a preferred embodiment, the one or more than one substance-binding domain within the vault-like particle is one or more than one polynucleotide-binding domain. In a preferred embodiment, the one or more than one polynucleotide-binding domain is a non-specific polynucleotide-binding peptide. In a preferred embodiment, the one or more than one polynucleotide-binding domain is a specific polynucleotide-binding peptide.

In another embodiment, the modified MVP of the vault-like particle comprising modified MVP comprises an amino acid sequence added to the N-terminal of the MVP creates a sensor in the vault-like particle. In one embodiment, the sensor is selected from the group consisting of a chemical sensor, an ionic sensor, a microorganism sensor, an optical sensor and a pH sensor. In one embodiment, the sensor is a green fluorescent protein.

In another embodiment, the modified MVP of the vault-like particle comprising modified MVP comprises an amino acid sequence added to the C-terminal of the MVP which results in one or more than one receptor-binding domain. In one embodiment, the one or more than one receptor-binding domain is between 1 and 95 receptor-binding domains. In another embodiment, the one or more than one receptor-binding domain is 96 receptor-binding domains. In another embodiment, the one or more than one receptor-binding domain is greater than 96 receptor-binding domains. In one embodiment, the one or more than one receptor-binding domain is non-specific. In another embodiment, the one or more than one receptor-binding domain is specific.

In another embodiment, the modified MVP further comprises an amino acid sequence added to the C-terminal of the MVP which results in one or more than one receptor-binding domain. In one embodiment, the one or more than one receptor-binding domain is between 1 and 95 receptor-binding domains. In another embodiment, the one or more than one receptor-binding domain is 96 receptor-binding domains. In another embodiment, the one or more than one receptor-binding domain, is greater than 96 receptor-binding domains. In one embodiment, the one or more than one receptor-binding domain is non-specific. In another embodiment, the one or more than one receptor-binding domain is specific.

In another embodiment, the modified MVP comprises both an amino acid sequence added to the C-terminal of the MVP and an amino acid sequence added to the N-terminal of the MVP.

According to another embodiment of the present invention, the vault-like particle comprises MVP or modified MVP, and further comprises VPARP or a portion of VPARP comprising at least about 150 consecutive residues of VPARP. In one embodiment, the portion of VPARP comprises residues from about residue 1562 to 1724 of human VPARP, SEQ ID NO:3. In another embodiment, the portion of VPARP comprises residues from about residue 1473 to 1724 of human VPARP, SEQ ID NO:3. In another embodiment, the VPARP or portion of VPARP is modified. In one embodiment, the modification comprises adding an amino acid sequence added to the C-terminal of the VPARP or portion of VPARP. In another embodiment, the modification comprises adding an amino acid sequence added to the N-terminal of the VPARP or portion of VPARP. In another embodiment, the modification comprises adding an amino acid sequence added to both the C-terminal and the N-terminal of the VPARP or portion of VPARP. In one embodiment, the modified MVP comprises an amino acid sequence added to the C-terminal of the MVP. In another embodiment, the modified MVP comprises an amino acid sequence added to the N-terminal of the MVP. In another embodiment, the modified MVP comprises both a peptide added to the C-terminal and a peptide added to the N-terminal.

Also disclosed is a method of preventing damage by one or more than one substance to an organism, to a specific tissue, to specific cells, or to an environmental medium, by sequestering the one or more than one substance within a vault-like particle. The method comprises providing vault-like particles, administering the vault-like particles to the organism, tissue, cells or environmental medium, and allowing the vault-like particles to sequester the one or more than one substance within the vault-like particles. The one or more than one substance may be a heavy metal selected from the group consisting of cadmium, copper, gold and mercury. The one or more than one substance may be a toxin selected from the group consisting of arsenate, dioxin, an organochlorine, a pentachlorophenol and a polychlorinated biphenyl.

Providing the vault-like particles may comprise expressing the vault-like particles in a eukaryotic organism.

Also disclosed is a method of delivering one or more than one substance to an organism, to a specific tissue, to specific cells, or to an environmental medium. The method comprises providing vault-like particles comprising the one or more than one substance, and administering the vault-like particles comprising the one or more than one substance to the organism, tissue, cells or environmental medium. The vault-like particles may comprise, consist essentially of or consist of a modified MVP in addition to the one or more than one substance. The vault-like particles may comprise a modified VPARP or modified portion of VPARP. The vault-like particles may comprise both a modified MVP according to the present invention, and a modified VPARP or modified portion of VPARP. The one or more than one substance may be selected from the group consisting of an enzyme, a pharmaceutical agent, a plasmid, a polynucleotide, a polypeptide, a sensor and a combination of the preceding. The one or more than one substance may be adenosine deaminase.

Also disclosed is a method of delivering one or more than one sensor to an organism, to a specific tissue, to specific cells, or to an environmental medium. The method comprises providing a vault-like particle comprising the one or more than one sensor and administering the vault-like particle to the organism, specific tissue, specific cells, or environmental medium. The vault-like particles may comprise, consist essentially of or consist of a modified MVP, in addition to the one or more than one sensor. The vault-like particles may comprise a modified VPARP or modified portion of VPARP. The vault-like particles may comprise both a modified MVP, and a modified VPARP or modified portion of VPARP. The sensor may be selected from the group consisting of a chemical sensor, a fluorescent sensor, an ionic sensor, a microorganism sensor, an optical sensor, and a pH sensor.

Also disclosed is a method of detecting a signal from a sensor within an organism, or a specific tissue or specific cells. The method comprises delivering one or more than one sensor to an organism, to a specific tissue, to specific cells, or to an environmental medium according to the present invention, and detecting the presence of the sensor. Detection may be accomplished by fluorometry or by spectrophotometry.

Also disclosed is a method of making vault-like particles. The method comprises creating polynucleotide sequences encoding one or more than one polypeptide selected from the group consisting of MVP, modified MVP, VPARP, a portion of VPARP, modified VPARP, a modified portion of VPARP, TEP1, a portion of TEP1, modified TEP1 and a modified portion of TEP1, using the polynucleotide sequences created to generate a bacmid DNA, using the bacmid DNA to generate a baculovirus comprising the sequence, and using the baculovirus to infect insect cells for protein production using an *in situ* assembly system.

Also disclosed is a method of making vault-like particles comprising one or more than one substance. The method comprises making vault-like particles as described above, and co-incubated the vault-like particles with the one or more than one substance. The one or more than one substance may be selected from the group consisting of enzyme, a pharmaceutical agent, a plasmid, a polynucleotide, a polypeptide, a sensor and a combination of the preceding. The method may further comprise purifying the vault-like particles after making the vault-like particles.

### DESCRIPTION

According to one embodiment of the present invention, there is provided a method of using vault-like particles as carrier molecules to deliver one or more than one substance to an environmental medium.

The disclosure also provides a vault-like particle useful as a carrier molecule for delivering one or more than one substance to a living system, such as an organism, specific tissue or specific cell, or to an environmental medium. The disclosure also provides a method of delivering one or more than one substance to an organism, or to a specific tissue or specific cells, or to an environmental medium. The method comprises providing vault-like particles comprising the substance, and administering the vault-like particles comprising the substance to the organism, tissue or cells, or to the environmental medium.

The disclosure also provides a method of delivering vault-like particles to a specific tissue or specific cells, or to an environmental medium. The method comprises providing vault-like particles having a receptor-binding domain on the surface of the vault-like particles, and administering the vault-like particles to the tissue or cells, or to the environmental medium.

The disclosure also provides a vault- like particle useful for sequestering the one or more than one substance within the vault-like particle. Also disclosed is a method of preventing damage by one or more than one substance to an organism, or to a specific tissue or specific cells, or to an environmental medium, by sequestering the one or more than one substance within a vault-like particle. The method comprises providing vault-like particles comprising one or more than one substance-binding domain within the vault-like particle, administering the vault-like particles to the organism, tissue or cells, or to the environmental medium, and allowing the vault-like particles to sequester the one or more than one substance within the vault-like particles.

Advantageously, both vaults and vault-like particles are resistant to degradation, such as intracellular degradation or environmental degradation, and therefore, can be used to deliver substances to or to remove substances from both living and non-living systems. The embodiments of the present invention will now be disclosed in greater detail.

As used in this disclosure, "MVP," "VPARP" and "TEP1" means the full naturally occurring polypeptide sequence. "vRNA" means the full naturally occurring polynucleotide sequence. As will be appreciated by one of ordinary skill in the art with reference to this disclosure, the actual sequence of any of MVP, VPARP, TEP1 and vRNAs can be from any species suitable for the purposes disclosed in this disclosure, even though reference or examples are made to sequences from specific species. For example, when delivering substances to human organs or tissues, it is preferred to use human vaults or vault-like particles comprising human sequences for MVP, VPARP, TEP1 and vRNAs. Further, as will be appreciated by one of ordinary skill in the art with reference to this disclosure, there are some intraspecies variations in the sequences of MVP, VPARP, TEP1 and vRNAs that are not relevant to the purposes of the present invention. Therefore, references to MVP, VPARP, TEP1 and vRNAs are intended to include such intraspecies variants.

As used in this disclosure, the term "vault" or "vault particle," as compared to the term "vault-like particle" defined below, refers to a naturally occurring macro-molecular structure having MVP, VPARP, TEP1 and one or more than one vRNA, whether purified from natural sources or generated through recombinant technology.

As used in this disclosure, the term "vault-like particle" refers to a macro-molecular structure comprising any of the following:
1) MVP without VPARP, TEP1 and vRNA;
2) MVP and either VPARP or a portion of VPARP, without TEP1 and vRNA;
3) MVP and TEP1 or a portion of TEP1 with or without the one or more than one vRNA, and without VPARP;
4) MVP without VPARP, TEP1 and vRNA, where the MVP is modified to attract a specific substance within the vault-like particle, or modified to attract the vault-like particle to a specific tissue, cell type or environmental medium, or modified both to attract a specific substance within the vault-like particle and to attract the vault particle to a specific tissue, cell type or environmental medium; and
5) MVP, and either VPARP or a portion of VPARP, or TEP1 or a portion of TEP1 with or without the one or more than one vRNA, or with both VPARP or a portion of VPARP, and TEP1, with or without the one or more than one vRNA, where one or more than one of the MVP, VPARP or portion of VPARP and TEP1 is modified to attract a specific substance within the vault-like particle, or modified to attract the vault particle to a specific tissue, cell type or environmental medium, or modified both to attract a specific substance within the vault-like particle and to attract the vault particle to a specific tissue, cell type or environmental medium.

As used in this disclosure, the term "modified" and variations of the term, such as "modification," means one or more than one change to the naturally occurring sequence of MVP, VPARP or TEP1 selected from the group consisting of addition of a polypeptide sequence to the C-terminal, addition of a polypeptide sequence to the N-terminal, deletion of between about 1 and 100 amino acid residues from the C-terminal, deletion of between about 1 and 100 amino acid residues from the N-terminal, substitution of one or more than one amino acid residue that does not change the function of the polypeptide, as will be appreciated by one of ordinary skill in the art with reference to this disclosure, such as for example, an alanine to glycine substitution, and a combination of the preceding.

As used in this disclosure, the term "human" means *"Homo sapiens*."

As used in this disclosure, the terms "organism," "tissue" and "cell" include naturally occurring organisms, tissues and cells, genetically modified organisms, tissues and cells, and pathological tissues and cells, such as tumor cell lines *in vitro* and tumors *in vivo.*

As used in this disclosure, the term "environmental medium" means a non-living composition, composite, material, or mixture.

As used in this disclosure, the term "administering" includes any suitable route of administration, as will be appreciated by one of ordinary skill in the art with reference to this disclosure, including direct injection into a solid organ, direct injection into a cell mass such as a tumor, inhalation, intraperitoneal injection, intravenous injection, topical application on a mucous membrane, or application to or dispersion within an environmental medium, and a combination of the preceding. In one embodiment, the dosage of vaults or vault-like particles, with or without one or more than one substance enclosed within the vaults or vault-like particles, is between about 0.1 and 10,000 micrograms per kilogram of body weight or environmental medium. In another embodiment, the dosage of vaults or vault-like particles, with or without one or more than one substance enclosed within the vaults or vault-like particles, is between about 1 and 1,000 micrograms per kilogram of body weight or environmental medium. In another embodiment, the dosage of vaults or vault-like particles, with or without one or more than one substance enclosed within the vaults or vault-like particles, is between about 10 and 1,000 micrograms per kilogram of body weight or environmental medium. For intravenous injection and intraperitoneal injection, the dosage is preferably administered in a final volume of between about 0.1 and 10 ml. For inhalation the dosage is preferably administered in a final volume of between about 0.01 and 1 ml. As will be appreciated by one of ordinary skill in the art with reference to this disclosure, the dose can be repeated a one or more than one of times as needed using the same parameters to effect the purposes disclosed in this disclosure.

As used in this disclosure, "MS2" means the Enterobacteriophage MS2 coat protein, which is an RNA-binding protein that specifically binds a 21-nt RNA stem-loop with high affinity.

As used in this disclosure, the term "comprise" and variations of the term, such as "comprising" and "comprises," are not intended to exclude other additives, components, integers or steps.

The disclosure also provides a method of using naturally occurring vaults as carrier molecules to deliver one or more than one substance to an organism, or to a specific tissue or specific cells, or to an environmental medium. The method comprises, first, providing vaults. The vaults may be purified from natural sources, such as mammalian liver or spleen tissue, using methods known to those with skill in the art, such as for example tissue homogenization, differential centrifugation, discontinuous sucrose gradient fractionation and cesium chloride gradient fractionation. The vaults may be made using recombinant technology. Next, the one or more than one substance is incorporated into the provided vaults. Incorporation is preferably accomplished by incubating the vaults with the one or more than one substance at an appropriate temperature and for an appropriate time, as will be appreciated by one of ordinary skill in the art with reference to this disclosure. The vaults containing the one or more than one substance are then purified, such as for example sucrose gradient fractionation, as will be appreciated by one of ordinary skill in the art with reference to this disclosure. The one or more than one substance is preferably selected from the group consisting of an enzyme, a pharmaceutical agent, a plasmid, a polynucleotide, a polypeptide, a sensor and a combination of the preceding. Next, the vaults comprising the one or more than one substance are administered to an organism, to a specific tissue, to specific cells, or to an environmental medium. Administration is accomplished using any suitable route, as will be appreciated by one of ordinary skill in the art with reference to this disclosure.

Also disclosed is a vault-like particle useful as a carrier molecule for delivering one or more than one substance to an organism, to a specific tissue, to specific cells, or to an environmental medium, or useful for preventing damage by one or more than one substance to an organism, to a specific tissue, to specific cells, or to an environmental medium, by sequestering the one or more than one substance within a vault-like particle. The vault-like particle comprises MVP or modified MVP, and can further comprise VPARP or modified VPARP, a portion of VPARP or a modified portion of VPARP, and TEP1 or modified TEP1, a portion of TEP1 or a modified portion of TEP1 with or without the one or more than one vRNA. The modifications are preferably designed to attract a specific substance within the vault-like particle, to attract the vault-like particle to a specific tissue or cell type, or both to attract a specific substance within the vault-like particle and to attract the vault particle to a specific tissue or cell type.

In one embodiment, the MVP is human MVP, SEQ ID NO:1, GenBank accession number CAA56256, encoded by the cDNA, SEQ ID NO:2, GenBank accession number X79882. In another embodiment, the VPARP is human VPARP, SEQ ID NO:3, GenBank accession number AAD47250, encoded by the cDNA, SEQ ID NO:4, GenBank accession number AF158255. In another embodiment, the TEP1 is human TEP1, SEQ ID NO:5, GenBank accession number AAC51107, encoded by the cDNA, SEQ ID NO:6, GenBank: accession number U86136. In another embodiment, the vRNA is human vRNA, SEQ ID NO:7, GenBank accession number AF045143, SEQ ID NO:8, GenBank: accession number AF045144, or SEQ ID NO:9, GenBank: accession number AF045145, or a combination of the preceding.

In one embodiment, the MVP is *Rattus norvegicus* MVP, SEQ ID NO:10, GenBank accession number AAC52161, encoded by the cDNA, SEQ ID NO:11, GenBank: accession number U09870. In another embodiment, the TEP1 is *Rattus norvegicus* TEP1, SEQ ID NO:12, GenBank accession number AAB51690, encoded by the cDNA, SEQ ID NO:13, GenBank: accession number U89282. In another embodiment, the vRNA is *Rattus norvegicus* vRNA, SEQ ID NO:14, GenBank: accession number Z1171. As can be seen, *Rattus norvegicus* MVP and human MVP share over 90% homology.

The following disclosure of vault protein modifications references specific examples using specific human and *Rattus norvegicus* sequences of MVP, VPARP and TEP1 sequences, however, as will be appreciated by one of ordinary skill in the art with reference to this disclosure, corresponding modifications can be made using other sequences of these species and can be made using sequences from other species as appropriate for the disclosed purposes.

According to one embodiment of the present invention, the method uses a vault-like particle comprising, consisting essentially of, or consisting of modified MVP. In a preferred embodiment, the modification comprises adding an amino acid sequence to the N-terminal of the MVP which results in one or more than one substance-binding domain within the vault-like particle. When each copy of the MVP is modified in this manner, one or more than one of the substance-binding domains, such as 96 substance-binding domains, is present in each vault-like particle, however, vault-like particles can also be assembled from a mixture of MVP with the N-terminal modified and MVP without the N-terminal modified, to create vault-like particle with less than 96 substance-binding domains in the vault-like particle, and the added amino acid terminal sequences can be polymerized as will be appreciated by one of ordinary skill in the art with reference to this disclosure to create more than 96 substance binding domains in the vault-like particle.

In a preferred embodiment, the vault-like particle comprises, consists essentially of, or consists of an MVP modified by adding a peptide to the N-terminal to create a one or more than one of heavy metal binding domains. In a preferred embodiment, the heavy metal binding domains bind a heavy metal selected from the group consisting of cadmium, copper, gold and mercury. In a preferred embodiment, the peptide added to the N-terminal is a cysteine-rich peptide (CP), such as for example, SEQ ID NO: 15, the MVP is human MVP, SEQ ID NO:1, and the modification results in CP-MVP, SEQ ID NO: 16, encoded by the cDNA, SEQ ID NO: 17. In another preferred embodiment, the cysteine-rich peptide is SEQ ID NO:15, the MVP is *Rattus norvegicus* MVP, SEQ ID NO:10, and the modification results in CP-MVP, SEQ ID NO:18, encoded by the cDNA, SEQ ID NO: 19. These embodiments are particularly useful because vault-like particles consisting of CP-MVP, SEQ ID NO:16 or SEQ ID NO:18, are stable without the presence of other vault proteins.

In another embodiment, the vault-like particle comprises, consists essentially of, or consists of an MVP modified by adding a peptide to the N-terminal to create one or more than one polynucleotide-binding domain. In a preferred embodiment, the peptide is a non-specific polynucleotide-binding peptide, such as for example, HisT7, SEQ ID NO:20, encoded by the cDNA, SEQ ID NO:21, or a polylysine such as SEQ ID NO:22, encoded by the cDNA, SEQ ID NO:23, the MVP is human MVP, SEQ ID NO:1, and the modification results in HisT7-MVP, SEQ ID NO:24, encoded by the cDNA, SEQ ID NO:25, or in polylysine-MVP, SEQ ID NO:26, encoded by the cDNA, SEQ ID NO:27, respectfully. In another preferred embodiment, the peptide is a non-specific polynucleotide-binding peptide, such as for example, HisT7, SEQ ID NO:20, encoded by the cDNA, SEQ ID NO:21, or a polylysine such as SEQ ID NO:22, encoded by the cDNA, SEQ ID NO:23, the MVP is *Rattus norvegicus* MVP, SEQ ID NO:10, and the modification results in HisT7- MVP, SEQ ID NO:28, encoded by the cDNA, SEQ ID NO:29, or in polylysine-MVP, SEQ ID NO:30, encoded by the cDNA, SEQ ID NO:31, respectfully. HisT7-MVP, SEQ ID NO:24 and SEQ ID NO:28, are examples of modified MVP that can also be used to bind specific antibodies within the vault-like particle, in these cases, the T7 monoclonal antibody, but corresponding modifications can be made to bind other specific antibodies, as will be appreciated by one of ordinary skill in the art with reference to this disclosure. In another preferred embodiment, the peptide is a specific DNA binding peptide, such as for example, GAL4, SEQ ID NO:32, encoded by the cDNA, SEQ ID NO:33, the MVP is human MVP, SEQ ID NO:1, and the modification results in GAL4-MVP, SEQ ID NO:34, encoded by the cDNA, SEQ ID NO:35. In another preferred embodiment, the peptide is a specific DNA binding peptide, such as for example, GAL4, SEQ ID NO:32; encoded by the cDNA, SEQ ID NO:33, the MVP is *Rattus norvegicus* MVP, SEQ ID NO:10, and the modification results in GAL4-MVP, SEQ ID NO:36, encoded by the cDNA, SEQ ID NO:37. In another preferred embodiment, the peptide is a specific RNA binding peptide, such as for example, MS2, SEQ ID NO:38, encoded by the cDNA, SEQ ID NO:39, the MVP is human MVP, SEQ ID NO:1, and the modification results in MS2-MVP, SEQ ID NO:40, encoded by the cDNA, SEQ ID NO:41. In another preferred embodiment, the peptide is an RNA binding peptide, such as for example, MS2, SEQ ID NO:38, encoded by the cDNA, SEQ ID NO:39, the MVP is *Rattus norvegicus* MVP, SEQ ID NO:10, and the modification results in MS2-MVP, SEQ ID NO:42, encoded by the cDNA, SEQ ID NO:43.

In another embodiment, the vault-like particle comprises, consists essentially of, or consists of an MVP modified by adding a peptide to the N-terminal to create a sensor in the vault-like particle. The sensor can be any suitable sensor, as will be appreciated by one of ordinary skill in the art with reference to this disclosure, such as for example, a chemical sensor such as a cyclic-AMP binding protein, an ionic sensor such as a calcium or potassium sensor, a microorganism sensor such an antibody specific for *E. coli,* an optical sensor such as a quantum dot, and a pH sensor such as green fluorescence protein. In a preferred embodiment, the sensor is a fluorescent protein, such as green fluorescent protein (GL), SEQ ID NO:44, encoded by the cDNA, SEQ ID NO:45, the MVP is human MVP, SEQ ID NO:1, and the modification results in GL-MVP, SEQ ID NO:46, encoded by the cDNA, SEQ ID NO:47. In another preferred embodiment, the sensor is a fluorescent protein, such as green fluorescent protein (GL), SEQ ID NO:44, encoded by the cDNA, SEQ ID NO:45, the MVP is *Rattus norvegicus* MVP, SEQ ID NO:10, and the modification results in GL-MVP, SEQ ID NO:48, encoded by the cDNA, SEQ ID NO:49.

In another embodiment, the vault-like particle comprises MVP or modified MVP, and further comprises VPARP or a portion of VPARP comprising at least about 150 consecutive residues of VPARP, and modified by adding a peptide to either the C-terminal or the N-terminal to create a one or more than one of substance-binding domains or a one or more than one of sensors within the vault-like particles having the same purposes as disclosed with reference to modified MVP in this disclosure. By way of example only, in one embodiment, the residues are from about residue 1562 to residue 1724 of human VPARP, SEQ ID NO:3. In another embodiment, the residues are from about residue 1473 to residue 1724 of human VPARP, SEQ ID NO:3. The substance-binding domains on the VPARP or portion of VPARP serve the same functions as disclosed in this disclosure with respect to N-terminal modifications of MVP. For example, in one embodiment, the vault-like particles comprise residues 1473-1724 of VPARP, SEQ ID NO:3, modified by adding CP, SEQ ID NO:15, to the N-terminal, to create (1473-1724)CP-VPARP, SEQ ID NO:50, encoded by the cDNA, SEQ ID NO:51. In another embodiment, the vault-like particles comprise VPARP, SEQ ID NO:3, modified by adding CP, SEQ ID NO:15, to the N-terminal, to create CP-VPARP, SEQ ID NO:52, encoded by the cDNA, SEQ ID NO:53. In one embodiment, the vault-like particles comprise residues 1473-1724 of VPARP, SEQ ID NO:3, modified by adding GAL4, SEQ ID NO:32, to the N-terminal, to create GAL4-(1473-1724)VPARP, SEQ ID NO:54, encoded by the cDNA, SEQ ID NO:55. In another embodiment, the vault-like particles comprise VPARP, SEQ ID NO:3, modified by adding GAL4, SEQ ID NO:32, to the N-terminal, to create GAL4-VPARP, SEQ ID NO:56, encoded by the cDNA, SEQ ID NO:57. In another embodiment, the vault-like particles comprise residues 1473-1724 of VPARP, SEQ ID NO:3, modified by adding GL, SEQ ID NO:44, to the N-terminal, to create GL-(1473-1724)VPARP, SEQ ID NO:58, encoded by the cDNA, SEQ ID NO:59. In another embodiment, the vault-like particles comprise VPARP, SEQ ID NO:3, modified by adding GL, SEQ ID NO:44, to the N-terminal, to create GL-VPARP, SEQ ID NO:60, encoded by the cDNA, SEQ ID NO:61. In another embodiment, the vault-like particles comprise residues 1473-1724 of VPARP, SEQ ID NO:3, modified by adding MS2, SEQ ID NO:38, to the N-terminal, to create MS2-(1473-1724)VPARP, SEQ ID NO:62, encoded by the cDNA, SEQ ID NO:63. In another embodiment, the vault-like particles comprise VPARP, SEQ ID NO:3, modified by adding MS2, SEQ ID NO:38, to the N-terminal, to create MS2-VPARP, SEQ ID NO:64, encoded by the cDNA, SEQ ID NO:65. In another embodiment, the vault-like particles comprise residues 1473-1724 of VPARP, SEQ ID NO:3, modified by adding a *Photinus pyralis* luciferase (LUC), SEQ ID NO:66 GenBank accession number P08659, encoded by the pGL3-Basic vector SEQ ID NO:67, GenBank accession number U47295 to the N-terminal, to create LUC-(1473-1724)VPARP, SEQ ID NO:68, encoded by the cDNA, SEQ ID NO:69. In another embodiment, the vault-like particles comprise VPARP, SEQ ID NO:3, modified by adding LUC, SEQ ID NO:66, to the N-terminal, to create LUC-VPARP, SEQ ID NO:71, encoded by the cDNA, SEQ ID NO:72. Further, as will be appreciated by one of ordinary skill in the art with reference to this disclosure, the present invention also includes corresponding modifications to the C-terminal of VPARP or a portion of VPARP, and serve the same function. In a preferred embodiment, the substance binding domain binds the enzyme adenosine deaminase.

According to one embodiment of the present invention, the vault-like particle comprises, consists essentially of, or consists of MVP modified by adding an amino acid sequence to the C-terminal of the MVP which results in one or more than one receptor-binding domain, such as a protein targeting domain, on the surface of the vault-like particle. When each copy of the MVP is modified in this manner, one or more than one of the receptor-binding domains, such as 96 receptor-binding domains, is present on each vault-like particle, however, vault-like particles can also be assembled from a mixture of MVP with the C-terminal modified and MVP without the C-terminal modified, to create vault-like particle with less than 96 receptor-binding domains on the vault-like particle.

In a preferred embodiment, the vault-like particle comprises, consists essentially of, or consists of an MVP modified by adding a peptide to the C- terminal to create a one or more than one of eukaryotic cell receptor-binding domains on the exterior of the vault-like particles. In a preferred embodiment, the eukaryotic cell receptor-binding domain is generally non-specific. For example, in one embodiment, the peptide is Antennapedia (ANT), SEQ ID NO:72, encoded by the cDNA, SEQ ID NO:73, the MVP is human MVP, SEQ ID NO:1, and the modification results in MVP-ANT, SEQ ID NO:74, encoded by the cDNA, SEQ ID NO:75. In another embodiment, the peptide is ANT, SEQ ID NO:72, encoded by the cDNA, SEQ ID NO:73, the MVP is *Rattus norvegicus* MVP, SEQ ID NO:10, and the modification results in MVP-ANT, SEQ ID NO:76, encoded by the cDNA, SEQ ID NO:77. In another embodiment, the peptide is HIV-Tat (TAT), SEQ ID NO:78, encoded by the cDNA, SEQ ID NO:79, the MVP is human MVP, SEQ ID NO:1, and the modification results in MVP-TAT, SEQ ID NO:80, encoded by the cDNA, SEQ ID NO:81. In another embodiment, the peptide is TAT, SEQ ID NO:78, encoded by the cDNA, SEQ ID NO:79, the MVP is *Rattus norvegicus* MVP, SEQ ID NO:10, and the modification results in MVP-TAT, SEQ ID NO:82, encoded by the cDNA, SEQ ID NO:83. In another embodiment, the eukaryotic cell receptor-binding domain is specific to a certain type of eukaryotic cell receptor, such as for example a carcinoembryonic antigen receptor, a protein found on the surface of about 50% of all human tumors, or an epidermal growth factor (EGF) receptor. For example, in one embodiment, the peptide is anti-CEA scFv diabody (αCEA), SEQ ID NO:84, encoded by the cDNA, SEQ ID NO:85, the MVP is human MVP, SEQ ID NO:1, and the modification results in MVP-αCEA, SEQ ID NO:86, encoded by the cDNA, SEQ ID NO:87. In another embodiment, the peptide is αCEA, SEQ ID NO:84, encoded by the cDNA, SEQ ID NO:85, the MVP is *Rattus norvegicus* MVP, SEQ ID NO:10, and the modification results in MVP-αCEA, SEQ ID NO:88, encoded by the cDNA, SEQ ID NO:89. In another embodiment, the peptide is EGF, SEQ ID NO:90, encoded by the cDNA, SEQ ID NO:91, the MVP is human MVP, SEQ ID NO:1, and the modification results in MVP-EGF, SEQ ID NO:92, encoded by the cDNA, SEQ ID NO:93. In another embodiment, the peptide is EGF, SEQ ID NO:90, encoded by the cDNA, SEQ ID NO:91, the MVP is *Rattus norvegicus* MVP, SEQ ID NO:10, and the modification results in MVP-EGF, SEQ ID NO:94, encoded by the cDNA, SEQ ID NO:95.

According to one embodiment of the present invention, the vault-like particle comprises, consists essentially of, or consists of MVP modified by adding an amino acid sequence to the N-terminal and also modified by adding an amino acid sequence to the C-terminal. The modification of the N-terminal and the modification of the C-terminal can be any modification as disclosed in this disclosure, for the same purposes as disclosed in this disclosure. For example, the modification of the N-terminal can result in a substance-binding domain, such as for example a heavy metal binding domain or a polynucleotide binding domain, or can result in a sensor within the vault-like particle. The modification of the C-terminal can result in one or more than one receptor-binding domain on the surface of the vault-like particle. By way of example only, in one embodiment, the vault-like particle comprises, consists essentially of, or consists of MVP modified by adding GAL4, SEQ ID NO:32, to the N-terminal of human MVP, SEQ ID NO:1, and ANT, SEQ ID NO:72 to the C-terminal of human MVP, SEQ ID NO:1, to create GAL4-MVP-ANT, SEQ ID NO:96, encoded by the cDNA, SEQ ID NO:97. In another embodiment, the vault-like particle comprises, consists essentially of, or consists of MVP modified by adding GAL4, SEQ ID NO:32, to the N-terminal of *Rattus norvegicus* MVP, SEQ ID NO:10, and ANT, SEQ ID NO:72 to the C-terminal of *Rattus norvegicus* MVP, SEQ ID NO:10, to create GAL4-MVP-ANT, SEQ ID NO:98, encoded by the cDNA, SEQ ID NO:99. In another embodiment, the vault-like particle comprises, consists essentially of, or consists of MVP modified by adding GAL4, SEQ ID NO:32, to the N-terminal of human MVP, SEQ ID NO:1, and αCEA, SEQ ID NO:84 to the C-terminal of human MVP, SEQ ID NO:1, to create GAL4-MVP-αCEA, SEQ ID NO:100, encoded by the cDNA, SEQ ID NO:101. In another embodiment, the vault-like particle comprises, consists essentially of, or consists of MVP modified by adding GAL4, SEQ ID NO:32, to the N-terminal of *Rattus norvegicus* MVP, SEQ ID NO:10, and αCEA, SEQ ID NO:84 to the C-terminal of *Rattus norvegicus* MVP, SEQ ID NO:10, to create GAL4-MVP-αCEA, SEQ ID NO:102, encoded by the cDNA, SEQ ID NO:103. In another embodiment, the vault-like particle comprises, consists essentially of, or consists of MVP modified by adding GAL4, SEQ ID NO:32, to the N-terminal of human MVP, SEQ ID NO:1, and EGF, SEQ ID NO:90 to the C-terminal of human MVP, SEQ ID NO:1, to create GAL4-MVP-EGF, SEQ ID NO:104, encoded by the cDNA, SEQ ID NO:105. In another embodiment, the vault-like particle comprises, consists essentially of, or consists of MVP modified by adding GAL4, SEQ ID NO:32, to the N-terminal of *Rattus norvegicus* MVP, SEQ ID NO:10, and EGF, SEQ ID NO:90 to the C-terminal of *Rattus norvegicus* MVP, SEQ ID NO:10, to create GAL4-MVP-EGF, SEQ ID NO:106, encoded by the cDNA, SEQ ID NO:107. In another embodiment, the vault-like particle comprises, consists essentially of, or consists of MVP modified by adding GAL4, SEQ ID NO:32, to the N-terminal of human MVP, SEQ ID NO:1, and TAT, SEQ ID NO:78 to the C-terminal of human MVP, SEQ ID NO:1, to create GAL4-MVP-TAT, SEQ ID NO:108, encoded by the cDNA, SEQ ID NO:109. In another embodiment, the vault-like particle comprises, consists essentially of, or consists of MVP modified by adding GAL4, SEQ ID NO:32, to the N-terminal of *Rattus norvegicus* MVP, SEQ ID NO:1, and TAT, SEQ ID NO:78 to the C-terminal of *Rattus norvegicus* MVP, SEQ ID NO:10, to create GAL4-MVP-TAT, SEQ ID NO:110, encoded by the cDNA, SEQ ID NO:111. In one embodiment, the vault-like particle comprises, consists essentially of, or consists of MVP modified by adding MS2, SEQ ID NO:38, to the N-terminal of human MVP, SEQ ID NO:1, and ANT, SEQ ID NO:72 to the C-terminal of human MVP, SEQ ID NO:1, to create MS2-MVP-ANT, SEQ ID NO: 112, encoded by the cDNA, SEQ ID NO:113. In another embodiment, the vault-like particle comprises, consists essentially of, or consists of MVP modified by adding MS2, SEQ ID N0:38, to the N-terminal of *Rattus norvegicus* MVP, SEQ ID NO:10, and ANT, SEQ ID NO:72 to the C-terminal of *Rattus norvegicus* MVP, SEQ ID NO:10, to create MS2-MVP-ANT, SEQ ID NO:114, encoded by the cDNA, SEQ ID NO:115. In another embodiment, the vault-like particle comprises, consists essentially of, or consists of MVP modified by adding MS2, SEQ ID NO:38, to the N-terminal of human MVP, SEQ ID NO:1, and αCEA, SEQ ID NO:84 to the C-terminal of human MVP, SEQ ID NO:1, to create MS2-MVP-αCEA, SEQ ID NO:116, encoded by the cDNA, SEQ ID NO:117. In another embodiment, the vault-like particle comprises, consists essentially of, or consists of MVP modified by adding MS2, SEQ ID NO:38, to the N-terminal of *Rattus norvegicus* MVP, SEQ ID NO:10, and αCEA, SEQ ID NO:84 to the C-terminal of *Rattus norvegicus* MVP, SEQ ID NO:10, to create MS2-MVP-αCEA, SEQ ID NO:118, encoded by the cDNA, SEQ ID NO:119. In another embodiment, the vault-like particle comprises, consists essentially of, or consists of MVP modified by adding MS2, SEQ ID NO:38, to the N-terminal of human MVP, SEQ ID NO:1, and EGF, SEQ ID N0:90 to the C-terminal of human MVP, SEQ ID NO:1, to create MS2-MVP-EGF, SEQ ID NO:120, encoded by the cDNA, SEQ ID NO:121. In another embodiment, the vault-like particle comprises, consists essentially of, or consists of MVP modified by adding MS2, SEQ ID NO:38, to the N-terminal of *Rattus norvegicus* MVP, SEQ ID NO:10, and EGF, SEQ ID NO:90 to the C-terminal of *Rattus norvegicus* MVP, SEQ ID NO:10, to create MS2-MVP-EGF, SEQ ID NO:122, encoded by the cDNA, SEQ ID NO:123. In another embodiment, the vault-like particle comprises, consists essentially of, or consists of MVP modified by adding MS2, SEQ ID NO:38, to the N-terminal of human MVP, SEQ ID NO:1, and TAT, SEQ ID NO:78 to the C-terminal of human MVP, SEQ ID NO:1, to create MS2-MVP-TAT, SEQ ID NO:124, encoded by the cDNA, SEQ ID NO:125. In another embodiment, the vault-like particle comprises, consists essentially of, or consists of MVP modified by adding MS2, SEQ ID NO:38, to the N-terminal of *Rattus norvegicus* MVP, SEQ ID NO:1, and TAT, SEQ ID NO:78 to the C-terminal of *Rattus norvegicus* MVP, SEQ ID NO:10, to create MS2-MVP-TAT, SEQ ID NO:126, encoded by the cDNA, SEQ ID NO:127. In one embodiment, the vault-like particle comprises, consists essentially of, or consists of MVP modified by adding polylysine, SEQ ID NO:22, to the N-terminal of human MVP, SEQ ID NO:1, and ANT, SEQ ID NO:72 to the C-terminal of human MVP, SEQ ID NO:1, to create polylysine-MVP-ANT, SEQ ID NO:128, encoded by the cDNA, SEQ ID NO:129. In another embodiment, the vault-like particle comprises, consists essentially of, or consists of MVP modified by adding polylysine, SEQ ID N0:22, to the N-terminal of *Rattus norvegicus* MVP, SEQ ID NO: 10, and ANT, SEQ ID NO:72 to the C-terminal of *Rattus norvegicus* MVP, SEQ ID NO: 10, to create polylysine-MVP-ANT, SEQ ID NO:130, encoded by the cDNA, SEQ ID NO:131. In another embodiment, the vault-like particle comprises, consists essentially of, or consists of MVP modified by adding polylysine, SEQ ID NO:22, to the N-terminal of human MVP, SEQ ID NO:1, and αCEA, SEQ ID NO:84 to the C-terminal of human MVP, SEQ ID NO:1, to create polylysine-MVP-αCEA, SEQ ID NO:132, encoded by the cDNA, SEQ ID NO:133. In another embodiment, the vault-like particle comprises, consists essentially of, or consists of MVP modified by adding polylysine, SEQ ID NO:22, to the N-terminal of *Rattus norvegicus* MVP, SEQ ID NO:10, and αCEA, SEQ ID NO:84 to the C-terminal of *Rattus norvegicus* MVP, SEQ ID NO:10, to create polylysine-MVP-αCEA, SEQ ID NO:134, encoded by the cDNA, SEQ ID NO:135. In another embodiment, the vault-like particle comprises, consists essentially of, or consists of MVP modified by adding polylysine, SEQ ID NO:22, to the N-terminal of human MVP, SEQ ID NO:1, and EGF, SEQ ID NO:90 to the C-terminal of human MVP, SEQ ID NO:1, to create polylysine-MVP-EGF, SEQ ID NO:136, encoded by the cDNA, SEQ ID NO:137. In another embodiment, the vault-like particle comprises, consists essentially of, or consists of MVP modified by adding polylysine, SEQ ID NO:22, to the N-terminal of *Rattus norvegicus* MVP, SEQ ID NO:10, and EGF, SEQ ID NO:90 to the C-terminal of *Rattus norvegicus* MVP, SEQ ID NO:10, to create polylysine-MVP-EGF, SEQ ID NO:138, encoded by the cDNA, SEQ ID NO:139. In another embodiment, the vault-like particle comprises, consists essentially of, or consists of MVP modified by adding polylysine, SEQ ID NO:22, to the N-terminal of human MVP, SEQ ID NO:1, and TAT, SEQ ID NO:78 to the C-terminal of human MVP, SEQ ID NO:1, to create polylysine-MVP-TAT, SEQ ID NO:140, encoded by the cDNA, SEQ ID NO:141. In another embodiment, the vault-like particle comprises, consists essentially of, or consists of MVP modified by adding polylysine, SEQ ID NO:22, to the N-terminal of *Rattus norvegicus* MVP, SEQ ID NO:1, and TAT, SEQ ID NO:78 to the C-terminal of *Rattus norvegicus* MVP, SEQ ID NO:10, to create polylysine-MVP-TAT, SEQ ID NO:142, encoded by the cDNA, SEQ ID NO:143.

According to another embodiment of the present invention, the vault-like particle comprises MVP and VPARP or a portion of VPARP, where the MVP is modified by adding an amino acid sequence to the N-terminal or is modified by adding an amino acid sequence to the C-terminal, or is modified both by adding an amino acid sequence to the N-terminal and by adding an amino acid sequence to the C-terminal, and where the VPARP or portion of VPARP is modified by adding an amino acid sequence to the N-terminal or is modified by adding an amino acid sequence to the C-terminal, or is modified both by adding an amino acid sequence to the N-terminal and by adding an amino acid sequence to the C-terminal. The modifications can be any modification as disclosed in this disclosure, for the same purposes as disclosed in this disclosure.

The disclosure also provides a method of preventing damage by one or more than one substance to an organism, to a specific tissue, to specific cells, or to an environmental medium, by sequestering the one or more than one substance within a vault-like particle. The method comprises providing vault-like particles according to the present invention. The method further comprises administering the vault-like particles to the organism, tissue, cells or environmental medium, and allowing the vault-like particles to sequester the one or more than one substance within the vault-like particles. In one embodiment, the vault-like particles comprise, consist essentially of or consist of a modified MVP according to the present invention. The vault-like particles may comprise a modified VPARP or portion of VPARP according to the present invention. The vault-like particles may comprise both a modified MVP according to the present invention, and a modified VPARP or portion of VPARP according to the present invention. The vault-like particles preferably comprise, consist essentially of or consist of MVP modified by adding a peptide to the N-terminal to create a one or more than one of heavy metal binding domains. The one or more than one substance may be a heavy metal selected from the group consisting of cadmium, copper, gold and mercury. The one or more than one substance may be a toxin selected from the group consisting of arsenate, dioxin, an organochlorine, a pentachlorophenol and a polychlorinated biphenyl. The providing step preferably comprises expressing the vault-like particles in a eukaryotic organisms, such as for example an Acanthomoeba sp., yeast or *Dictostelium discoidieum*, capable of proliferating in contaminated soil, and the administering step comprises introducing the organisms with the expressed vault-like particles into the contaminated soil. For example, vault-like particles comprising an arsenate reductase enzyme within the vault-like particles can be expressed in the organisms and used to detoxify soil. For example, in one embodiment, modified MVP is provided comprising one or more than one arsenate-binding domain at the N-terminal. Arsenate reductase enzyme is cloned with residues 1473-1724 of human VPARP, SEQ ID NO:3 at either the C-terminal or the N-terminal. Both proteins are co-expressed in a primitive eukaryotic organisms, such as acanthomoeba, yeast or *Dictostelium discoidieum*, capable of proliferating in contaminated soil. The organisms engineered to contain the two modified proteins are introduced into contaminated soil, where they are exposed to the environmental toxin, such as arsenate. The expressed vault-like particles, comprising 96 or more copies of the arsenate-binding domain and the detoxification enzyme, arsenate reductase within the vault-like particles, then sequester and detoxify the environmental toxin, arsenate in the environmental medium.

The disclosure also provides a method of delivering one or more than one substance to an organism, to a specific tissue, to specific cells, or to an environmental medium. The method comprises providing vault-like particles according to the present invention comprising the one or more than one substance. The method further comprises administering the vault-like particles comprising the one or more than one substance to the organism, tissue, cells or environmental medium. The vault-like particles may comprise, consist essentially of or consist of a modified MVP according to the present invention, in addition to the one or more than one substance. In another embodiment, the vault-like particles comprise a modified VPARP or modified portion of VPARP according to the present invention. The vault-like particles may comprise both a modified MVP according to the present invention, and a modified VPARP or modified portion of VPARP according to the present invention. The one or more than one substance may be selected from the group consisting of an enzyme, a pharmaceutical agent, a plasmid, a polynucleotide, a polypeptide, a sensor and a combination of the preceding. The substance is preferably adenosine deaminase.

In another embodiment of the present invention, there is provided a method of delivering one or more than one sensor to an environmental medium. The method comprises providing a vault-like particle comprising the one or more than one sensor and administering the vault-like particle to the organism, specific tissue, specific cells, or environmental medium. In one embodiment, the vault-like particles comprise, consist essentially of or consist of a modified MVP according to the present invention, in addition to the one or more than one sensor. In another embodiment, the vault-like particles comprise a modified VPARP or modified portion of VPARP according to the present invention. In another embodiment, the vault-like particles comprise both a modified MVP according to the present invention, and a modified VPARP or modified portion of VPARP according to the present invention. The sensor can be any suitable sensor, as will be appreciated by one of ordinary skill in the art with reference to this disclosure, such as for example, a chemical sensor such as a cyclic-AMP binding protein, an ionic sensor such as a calcium or potassium sensor, a microorganism sensor such an antibody specific for *E*. *coli,* an optical sensor such as a quantum dot, and a pH sensor such as green fluorescence protein. In a preferred embodiment, the sensor is a fluorescent sensor.

The disclosure also provides a method of detecting a signal from a sensor within an organism, or a specific tissue or specific cells. The method comprises delivering one or more than one sensor to an organism, to a specific tissue, to specific cells, or to an environmental medium, according to a method of the present invention. Then, the presence of the sensor is detected. Detection is performed using standard techniques, such as for example, fluorometry or spectrophotometry. This method can be used, for example, to determine the pH within cells, where the sensor is a pH dependent fluorescent sensor, as will be appreciated by one of ordinary skill in the art with reference to this disclosure.

The disclosure also provides a method of making vault-like particles according to the present invention. The method comprises creating polynucleotide sequences encoding one or more than one polypeptide selected from the group consisting of MVP, modified MVP, VPARP, a portion of VPARP, modified VPARP, a modified portion of VPARP, TEP1, a portion of TEP1, modified TEP1 and a modified portion of TEP1, using standard molecular biological procedures, such as polymerase chain reaction and specific oligonucleotides, as will be appreciated by one of ordinary skill in the art with reference to this disclosure. Preferably, the polynucleotide sequences are used to generate a bacmid DNA that is used to generate a baculovirus comprising the sequence. The baculovirus is then used to infect insect cells for protein production using an *in situ* assembly system, such as the baculovirus protein expression system, according to standard techniques, as will be appreciated by one of ordinary skill in the art with reference to this disclosure. Advantageously, we have used the baculovirus protein expression system to produce milligram quantities of vault-like particles, and this system can be scaled up to allow production of gram quantities of vault-like particles according to the present invention.

The disclosure also provides a method of making vault-like particles having one or more than one substance, such as an enzyme, a pharmaceutical agent, a plasmid, a polynucleotide, a polypeptide, a sensor and a combination of the preceding, within the vault-like particles. The method comprises making the vault-like particles according to a method of the present invention. Next, the vault-like particles are purified using, such as for example, standard procedures over sucrose gradients. Then, the vault-like particles are co-incubated with one or more than one substance, until the one or more than one substance equilibrates within the vault-like particles or until enough of the one or more than one substance is loaded in the vault-like particles for the intended purpose.

Although the present invention has been discussed in considerable detail with reference to certain preferred embodiments, other embodiments are possible. Therefore, the scope of the appended claims should not be limited to the description of preferred embodiments contained in this disclosure.

### SEQUENCE LISTING

<110> Regents of the University of California, The
   Leonard, Rome H.
   Valerie, Kickhoefer A.
   Sujna, Raval-Fernandes
   Phoebe, Stewart L.
<120> Vault and Vault-like Carrier Molecules
<130> 14399-1PCT
<140> to be assigned
   <141> 2004-03-10
<150> 60/453,800 <151> 2003-03-10
<160> 143
<170> PatentIn version 3.2
<210> 1
   <211> 893
   <212> PRT
   <213> Homo sapiens
<400> 1
   Met Ala Thr Glu Glu Phe Ile Ile Arg Ile Pro Pro Tyr His Tyr Ile
   1               5                   10                  15
   His Val Leu Asp Gln Asn Ser Asn Val Ser Arg Val Glu Val Gly Pro
               20                  25                  30
   Lys Thr Tyr Ile Arg Gln Asp Asn Glu Arg Val Leu Phe Ala Pro Met
           35                  40                  45
   Arg Met Val Thr Val Pro Pro Arg His Tyr Cys Thr Val Ala Asn Pro
       50                  55                  60
   Val Ser Arg Asp Ala Gln Gly Leu Val Leu Phe Asp Val Thr Gly Gln
   65                  70                  75                  80
   Val Arg Leu Arg His Ala Asp Leu Glu Ile Arg Leu Ala Gln Asp Pro
                   85                  90                  95
   Phe Pro Leu Tyr Pro Gly Glu Val Leu Glu Lys Asp Ile Thr Pro Leu
               100                 105                 110
   Gln Val Val Leu Pro Asn Thr Ala Leu His Leu Lys Ala Leu Leu Asp
           115                 120                 125
   Phe Glu Asp Lys Asp Gly Asp Lys Val Val Ala Gly Asp Glu Trp Leu
       130                 135                 140
   Phe Glu Gly Pro Gly Thr Tyr Ile Pro Arg Lys Glu Val Glu Val Val
   145                 150                 155                 160
   Glu Ile Ile Gln Ala Thr Ile Ile Arg Gln Asn Gln Ala Leu Arg Leu
                   165                 170                 175
   Arg Ala Arg Lys Glu Cys Trp Asp Arg Asp Gly Lys Glu Arg Val Thr
               180                 185                 190
   Gly Glu Glu Trp Leu Val Thr Thr Val Gly Ala Tyr Leu Pro Ala Val
           195                 200                 205
   Phe Glu Glu Val Leu Asp Leu Val Asp Ala Val Ile Leu Thr Glu Lys
       210                 215                 220
   Thr Ala Leu His Leu Arg Ala Arg Arg Asn Phe Arg Asp Phe Arg Gly
   225                 230                 235                 240
   Val Ser Arg Arg Thr Gly Glu Glu Trp Leu Val Thr Val Gln Asp Thr
                   245                 250                 255
   Glu Ala His Val Pro Asp Val His Glu Glu Val Leu Gly Val Val Pro
               260                 265                 270
   Ile Thr Thr Leu Gly Pro His Asn Tyr Cys Val Ile Leu Asp Pro Val
           275                 280                 285
   Gly Pro Asp Gly Lys Asn Gln Leu Gly Gln Lys Arg Val Val Lys Gly
           290                     295                     300
   Glu Lys Ser Phe Phe Leu Gln Pro Gly Glu Gln Leu Glu Gln Gly Ile
   305                 310                     315                 320
   Gln Asp Val Tyr Val Leu Ser Glu Gln Gln Gly Leu Leu Leu Arg Ala
                   325                     330                     335
   Leu Gln Pro Leu Glu Glu Gly Glu Asp Glu Glu Lys Val Ser His Gln
               340                     345                     350
   Ala Gly Asp His Trp Leu Ile Arg Gly Pro Leu Glu Tyr Val Pro Ser
           355                     360                     365
   Ala Lys Val Glu Val Val Glu Glu Arg Gln Ala Ile Pro Leu Asp Glu
       370                     375                     380
   Asn Glu Gly Ile Tyr Val Gln Asp Val Lys Thr Gly Lys Val Arg Ala
   385                 390                     395                 400
   Val Ile Gly Ser Thr Tyr Met Leu Thr Gln Asp Glu Val Leu Trp Glu
                   405                     410                     415
   Lys Glu Leu Pro Pro Gly Val Glu Glu Leu Leu Asn Lys Gly Gln Asp
               420                     425                     430
   Pro Leu Ala Asp Arg Gly Glu Lys Asp Thr Ala Lys Ser Leu Gln Pro
           435                     440                     445
   Leu Ala Pro Arg Asn Lys Thr Arg Val Val Ser Tyr Arg Val Pro His
       450                     455                     460
   Asn Ala Ala Val Gln Val Tyr Asp Tyr Arg Glu Lys Arg Ala Arg Val
   465                 470                     475                 480
   Val Phe Gly Pro Glu Leu Val Ser Leu Gly Pro Glu Glu Gln Phe Thr
                   485                     490                     495
   Val Leu Ser Leu Ser Ala Gly Arg Pro Lys Arg Pro His Ala Arg Arg
               500                     505                     510
   Ala Leu Cys Leu Leu Leu Gly Pro Asp Phe Phe Thr Asp Val Ile Thr
           515                     520                     525
   Ile Glu Thr Ala Asp His Ala Arg Leu Gln Leu Gln Leu Ala Tyr Asn
       530                     535                     540
   Trp His Phe Glu Val Asn Asp Arg Lys Asp Pro Gln Glu Thr Ala Lys
   545                 550                     555                 560
   Leu Phe Ser Val Pro Asp Phe Val Gly Asp Ala Cys Lys Ala Ile Ala
                   565                     570                     575
   Ser Arg Val Arg Gly Ala Val Ala Ser Val Thr Phe Asp Asp Phe His
               580                     585                     590
   Lys Asn Ser Ala Arg Ile Ile Arg Thr Ala Val Phe Gly Phe Glu Thr
           595                     600                     605
   Ser Glu Ala Lys Gly Pro Asp Gly Met Ala Leu Pro Arg Pro Arg Asp
       610                     615                     620
   Gln Ala Val Phe Pro Gln Asn Gly Leu Val Val Ser Ser Val Asp Val
   625                 630                     635                 640
   Gln Ser Val Glu Pro Val Asp Gln Arg Thr Arg Asp Ala Leu Gln Arg
                   645                     650                     655
   Ser Val Gln Leu Ala Ile Glu Ile Thr Thr Asn Ser Gln Glu Ala Ala
               660                     665                     670
   Ala Lys His Glu Ala Gln Arg Leu Glu Gln Glu Ala Arg Gly Arg Leu
           675                     680                     685
   Glu Arg Gln Lys Ile Leu Asp Gln Ser Glu Ala Glu Lys Ala Arg Lys
       690                     695                     700
   Glu Leu Leu Glu Leu Glu Ala Leu Ser Met Ala Val Glu Ser Thr Gly
   705                 710                     715                 720
   Thr Ala Lys Ala Glu Ala Glu Ser Arg Ala Glu Ala Ala Arg Ile Glu
                   725                     730                     735
   Gly Glu Gly Ser Val Leu Gln Ala Lys Leu Lys Ala Gln Ala Leu Ala
               740                     745                     750
   Ile Glu Thr Glu Ala Glu Leu Gln Arg Val Gln Lys Val Arg Glu Leu
           755                     760                     765
   Glu Leu Val Tyr Ala Arg Ala Gln Leu Glu Leu Glu Val Ser Lys Ala
       770                     775                     780
   Gln Gln Leu Ala Glu Val Glu Val Lys Lys Phe Lys Gln Met Thr Glu
   785                 790                     795                 800
   Ala Ile Gly Pro Ser Thr Ile Arg Asp Leu Ala Val Ala Gly Pro Glu
                   805                     810                     815
   Met Gln Val Lys Leu Leu Gln Ser Leu Gly Leu Lys Ser Thr Leu Ile
               820                 825                 830
   Thr Asp Gly Ser Thr Pro Ile Asn Leu Phe Asn Thr Ala Phe Gly Leu
           835                 840                 845
   Leu Gly Met Gly Pro Glu Gly Gln Pro Leu Gly Arg Arg Val Ala Ser
       850                 855                 860
   Gly Pro Ser Pro Gly Glu Gly Ile Ser Pro Gln Ser Ala Gln Ala Pro
   865                 870                 875                 880
   Gln Ala Pro Gly Asp Asn His Val Val Pro Val Leu Arg
                   885                 890
<210> SEQ ID NO 2
   <211> LENGTH: 2682
   <212> TYPE: DNA
   <213> ORGANISM: Homo sapiens
<400> SEQUENCE: 2
<210> SEQ ID NO 3
   <211> LENGTH: 1724
   <212> TYPE: PRT
   <213> ORGANISM: Homo sapiens
<400> SEQUENCE: 3
<210> SEQ ID NO 4
   <211> LENGTH: 5175
   <212> TYPE: DNA
   <213> ORGANISM: Homo sapiens
<400> SEQUENCE: 4
<210> SEQ ID NO 5
   <211> LENGTH: 2627
   <212> TYPE: PRT
   <213> ORGANISM: Homo sapiens
<400> SEQUENCE: 5
<210> SEQ ID NO 6
   <211> LENGTH: 7884
   <212> TYPE: DNA
   <213> ORGANISM: Homo sapiens
<400> SEQUENCE: 6
<210> SEQ ID NO 7
   <211> LENGTH: 98
   <212> TYPE: RNA
   <213> ORGANISM: Homo sapiens
<400> SEQUENCE: 7
<210> SEQ ID NO 8
   <211> LENGTH: 88
   <212> TYPE: RNA
   <213> ORGANISM: Homo sapiens
<400> SEQUENCE: 8
<210> SEQ ID NO 9
   <211> LENGTH: 88
   <212> TYPE: RNA
   <213> ORGANISM: Homo sapiens
<400> SEQUENCE: 9
<210> SEQ ID NO 10
   <211> LENGTH: 861
   <212> TYPE: PRT
   <213> ORGANISM: Rattus norvegicus
<400> SEQUENCE: 10
<210> SEQ ID NO 11
   <211> LENGTH: 2586
   <212> TYPE: DNA
   <213> ORGANISM: Rattus norvegicus
<400> SEQUENCE: 11
<210> SEQ ID NO 12
   <211> LENGTH: 2629
   <212> TYPE: PRT
   <213> ORGANISM: Rattus norvegicus
<400> SEQUENCE: 12
<210> SEQ ID NO 13
   <211> LENGTH: 7890
   <212> TYPE: DNA
   <213> ORGANISM: Rattus norvegicus
<400> SEQUENCE: 13
<210> SEQ ID NO 14
   <211> LENGTH: 143
   <212> TYPE: RNA
   <213> ORGANISM: Rattus norvegicus
<400> SEQUENCE: 14
<210> SEQ ID NO 15
   <211> LENGTH: 12
   <212> TYPE: PRT
   <213> ORGANISM: Artificial Sequence
<220> FEATURE:
   <223> OTHER INFORMATION: completely synthesized
<400> SEQUENCE: 15
<210> SEQ ID NO 16
   <211> LENGTH: 905
   <212> TYPE: PRT
   <213> ORGANISM: Artificial Sequence
<220> FEATURE:
   <223> OTHER INFORMATION: synthesized and Homo sapines
<400> SEQUENCE: 16
<210> SEQ ID NO 17
   <211> LENGTH: 2718
   <212> TYPE: DNA
   <213> ORGANISM: Artificial Sequence
<220> FEATURE:
   <223> OTHER INFORMATION: synthesized and Homo sapines
<400> SEQUENCE: 17
<210> SEQ ID NO 18
   <211> LENGTH: 873
   <212> TYPE: PRT
   <213> ORGANISM: Artificial Sequence
<220> FEATURE:
   <223> OTHER INFORMATION: synthesized and Rattus norvegicus
<400> SEQUENCE: 18
<210> SEQ ID NO 19
   <211> LENGTH: 2622
   <212> TYPE: DNA
   <213> ORGANISM: Artificial Sequence
<220> FEATURE:
   <223> OTHER INFORMATION: synthesized and Rattus norvegicus
<400> SEQUENCE: 19
<210> SEQ ID NO 20
   <211> LENGTH: 31
   <212> TYPE: PRT
   <213> ORGANISM: Artificial Sequence
<220> FEATURE:
   <223> OTHER INFORMATION: completely synthesized
<400> SEQUENCE: 20
<210> SEQ ID NO 21
   <211> LENGTH: 89
   <212> TYPE: DNA
   <213> ORGANISM: Artificial Sequence
<220> FEATURE:
   <223> OTHER INFORMATION: completely synthesized
<400> SEQUENCE: 21
<210> SEQ ID NO 22
   <211> LENGTH: 17
   <212> TYPE: PRT
   <213> ORGANISM: Artificial Sequence
<220> FEATURE:
   <223> OTHER INFORMATION: completely synthesized
<400> SEQUENCE: 22
<210> SEQ ID NO 23
   <211> LENGTH: 51
   <212> TYPE: DNA
   <213> ORGANISM: Artificial Sequence
<220> FEATURE:
   <223> OTHER INFORMATION: completely synthesized
<400> SEQUENCE: 23
<210> SEQ ID NO 24
   <211> LENGTH: 923
   <212> TYPE: PRT
   <213> ORGANISM: Artificial Sequence
<220> FEATURE:
   <223> OTHER INFORMATION: synthesized and Homo sapiens
<400> SEQUENCE: 24
<210> SEQ ID NO 25
   <211> LENGTH: 2772
   <212> TYPE: DNA
   <213> ORGANISM: Artificial Sequence
<220> FEATURE:
   <223> OTHER INFORMATION: synthesized and Homo sapiens
<400> SEQUENCE: 25
<210> SEQ ID NO 26
   <211> LENGTH: 910
   <212> TYPE: PRT
   <213> ORGANISM: Artificial Sequence
<220> FEATURE:
   <223> OTHER INFORMATION: synthesized and Homo sapiens
<400> SEQUENCE: 26
<210> SEQ ID NO 27
   <211> LENGTH: 2733
   <212> TYPE: DNA
   <213> ORGANISM: Artificial Sequence
<220> FEATURE:
   <223> OTHER INFORMATION: synthesized and Homo sapiens
<400> SEQUENCE: 27
<210> SEQ ID NO 28
   <211> LENGTH: 892
   <212> TYPE: PRT
   <213> ORGANISM: Artificial.Sequence
<220> FEATURE:
   <223> OTHER INFORMATION: synthesized and Rattus norvegicus
<400> SEQUENCE: 28
<210> SEQ ID NO 29
   <211> LENGTH: 2679
   <212> TYPE: DNA
   <213> ORGANISM: Artificial Sequence
<220> FEATURE:
   <223> OTHER INFORMATION: synthesized and Rattus norvegicus
<400> SEQUENCE: 29
<210> SEQ ID NO 30
   <211> LENGTH: 878
   <212> TYPE: PRT
   <213> ORGANISM: Artificial Sequence
<220> FEATURE:
   <223> OTHER INFORMATION: synthesized and Rattus norvegicus
<400> SEQUENCE: 30
<210> SEQ ID NO 31
   <211> LENGTH: 2637
   <212> TYPE: DNA
   <213> ORGANISM: Artificial Sequence
<220> FEATURE:
   <223> OTHER INFORMATION: synthesized and Rattus norvegicus
<400> SEQUENCE: 31
<210> SEQ ID NO 32
   <211> LENGTH: 96
   <212> TYPE: PRT
   <213> ORGANISM: Saccharomyces cerevisiae
<400> SEQUENCE: 32
<210> SEQ ID NO 33
   <211> LENGTH: 288
   <212> TYPE: DNA
   <213> ORGANISM: Saccharomyces cerevisiae
<400> SEQUENCE: 33
<210> SEQ ID NO 34
   <211> LENGTH: 989
   <212> TYPE: PRT
   <213> ORGANISM: Artificial Sequence
<220> FEATURE:
   <223> OTHER INFORMATION: Saccharomyces cerevisiae and Homo sapiens
<400> SEQUENCE: 34
<210> SEQ ID NO 35
   <211> LENGTH: 2970
   <212> TYPE: DNA
   <213> ORGANISM: Artificial Sequence
<220> FEATURE:
   <223> OTHER INFORMATION: Saccharomyces cerevisiae and Homo sapiens
<400> SEQUENCE: 35
<210> SEQ ID NO 36
   <211> LENGTH: 957
   <212> TYPE: PRT
   <213> ORGANISM: Artificial Sequence
<220> FEATURE:
   <223> OTHER INFORMATION: Saccharomyces cerevisiae and Rattus norvegicus
<400> SEQUENCE: 36
<210> SEQ ID NO 37
   <211> LENGTH: 2874
   <212> TYPE: DNA
   <213> ORGANISM: Artificial Sequence
<220> FEATURE:
   <223> OTHER INFORMATION: Saccharomyces cerevisiae and Rattus norvegicus
<400> SEQUENCE: 37
<210> SEQ ID NO 38
   <211> LENGTH: 130
   <212> TYPE: PRT
   <213> ORGANISM: Levivirus
<400> SEQUENCE: 38
<210> SEQ ID NO 39
   <211> LENGTH: 393
   <212> TYPE: DNA
   <213> ORGANISM: Levivirus
<400> SEQUENCE: 39
<210> SEQ ID NO 40
   <211> LENGTH: 1024
   <212> TYPE: PRT
   <213> ORGANISM: Artificial Sequence
<220> FEATURE:
   <223> OTHER INFORMATION: Levivirus and Homo sapiens
<400> SEQUENCE: 40
<210> SEQ ID NO 41
   <211> LENGTH: 3075
   <212> TYPE: DNA
   <213> ORGANISM: Artificial Sequence
<220> FEATURE:
   <223> OTHER INFORMATION: Levivirus and Homo sapiens
<400> SEQUENCE: 41
<210> SEQ ID NO 42
   <211> LENGTH: 992
   <212> TYPE: PRT
   <213> ORGANISM: Artificial Sequence
<220> FEATURE:
   <223> OTHER INFORMATION: Levivirus and Rattus norvegicus
<400> SEQUENCE: 42
<210> SEQ ID NO 43
   <211> LENGTH: 2979
   <212> TYPE: DNA
   <213> ORGANISM: Artificial Sequence
<220> FEATURE:
   <223> OTHER INFORMATION: Levivirus and Rattus norvegicus
<400> SEQUENCE: 43
<210> SEQ ID NO 44
   <211> LENGTH: 239
   <212> TYPE: PRT
   <213> ORGANISM: Artificial Sequence
<220> FEATURE:
   <223> OTHER INFORMATION: completely synthesized
<400> SEQUENCE: 44
<210> SEQ ID NO 45
   <211> LENGTH: 720
   <212> TYPE: DNA
   <213> ORGANISM: Artificial Sequence
<220> FEATURE:
   <223> OTHER INFORMATION: completely synthesized
<400> SEQUENCE: 45
<210> SEQ ID NO 46
   <211> LENGTH: 1132
   <212> TYPE: PRT
   <213> ORGANISM: Artificial Sequence
<220> FEATURE:
   <223> OTHER INFORMATION: synthesized and Homo spiens
<400> SEQUENCE: 46
<210> SEQ ID NO 47
   <211> LENGTH: 3399
   <212> TYPE: PRT
   <213> ORGANISM: Artificial Sequence
<220> FEATURE:
   <223> OTHER INFORMATION: synthesized and Homo sapiens
<400> SEQUENCE: 47
<210> SEQ ID NO 48
   <211> LENGTH: 1100
   <212> TYPE: PRT
   <213> ORGANISM: Artificial Sequence
<220> FEATURE:
   <223> OTHER INFORMATION: synthesized and Rattus norvegicus
<400> SEQUENCE: 48
<210> SEQ ID NO 49
   <211> LENGTH: 3303
   <212> TYPE: PRT
   <213> ORGANISM: Artificial Sequence
<220> FEATURE:
   <223> OTHER INFORMATION: synthesized and Rattus norvegicus
<400> SEQUENCE: 49
<210> SEQ ID NO 50
   <211> LENGTH: 267
   <212> TYPE: PRT
   <213> ORGANISM: Artificial Sequence
<220> FEATURE:
   <223> OTHER INFORMATION: synthesized and Homo sapiens
<400> SEQUENCE: 50
<210> SEQ ID NO 51
   <211> LENGTH: 804
   <212> TYPE: DNA
   <213> ORGANISM: Artificial Sequence
<220> FEATURE:
   <223> OTHER INFORMATION: synthesized and Homo sapiens
<400> SEQUENCE: 51
<210> SEQ ID NO 52
   <211> LENGTH: 1736
   <212> TYPE: PRT
   <213> ORGANISM: Artificial Sequence
<220> FEATURE:
   <223> OTHER INFORMATION: synthesized and Homo sapiens
<400> SEQUENCE: 52
<210> SEQ ID NO 53
   <211> LENGTH: 5211
   <212> TYPE: DNA
   <213> ORGANISM: Artificial Sequence
<220> FEATURE:
   <223> OTHER INFORMATION: synthesized and Homo sapiens
<400> SEQUENCE: 53
<210> SEQ ID NO 54
   <211> LENGTH: 351
   <212> TYPE: PRT
   <213> ORGANISM: Artificial Sequence
<220> FEATURE:
   <223> OTHER INFORMATION: Saccaromyces cerevisiae and Homo sapiens
<400> SEQUENCE: 54
<210> SEQ ID NO 55
   <211> LENGTH: 1056
   <212> TYPE: DNA
   <213> ORGANISM: Artificial Sequence
<220> FEATURE:
   <223> OTHER INFORMATION: Saccaromyces cerevisiae and Homo sapiens
<400> SEQUENCE: 55
<210> SEQ ID NO 56
   <211> LENGTH: 1820
   <212> TYPE: PRT
   <213> ORGANISM: Artificial Sequence
<220> FEATURE:
   <223> OTHER INFORMATION: Saccaromyces cerevisiae and Homo sapiens
<400> SEQUENCE: 56
<210> SEQ ID NO 57
   <211> LENGTH: 5463
   <212> TYPE: DNA
   <213> ORGANISM: Artificial Sequence
<220> FEATURE:
   <223> OTHER INFORMATION: Saccaromyces cerevisiae and Homo sapiens
<400> SEQUENCE: 57
<210> SEQ ID NO 58
   <211> LENGTH: 491
   <212> TYPE: PRT
   <213> ORGANISM: Artificial Sequence
<220> FEATURE:
   <223> OTHER INFORMATION: synthesized and Homo sapiens
<400> SEQUENCE: 58
<210> SEQ ID NO 59
   <211> LENGTH: 1476
   <212> TYPE: DNA
   <213> ORGANISM: Artificial Sequence
<220> FEATURE:
   <223> OTHER INFORMATION: synthesized and Homo sapiens
<400> SEQUENCE: 59
<210> SEQ ID NO 60
   <211> LENGTH: 1961
   <212> TYPE: PRT
   <213> ORGANISM: Artificial Sequence
<220> FEATURE:
   <223> OTHER INFORMATION: synthesized and Homo sapiens
<400> SEQUENCE: 60
<210> SEQ ID NO 61
   <211> LENGTH: 5889
   <212> TYPE: DNA
   <213> ORGANISM: Artificial Sequence
<220> FEATURE:
   <223> OTHER INFORMATION: synthesized and Homo sapiens
<400> SEQUENCE: 61
<210> SEQ ID NO 62
   <211> LENGTH: 385
   <212> TYPE: PRT
   <213> ORGANISM: Artificial Sequence
<220> FEATURE:
   <223> OTHER INFORMATION: Levivirus and Homo sapiens
<400> SEQUENCE: 62
<210> SEQ ID NO 63
   <211> LENGTH: 1158
   <212> TYPE: DNA
   <213> ORGANISM: Artificial Sequence
<220> FEATURE:
   <223> OTHER INFORMATION: Levivirus and Homo sapiens
<400> SEQUENCE: 63
<210> SEQ ID NO 64
   <211> LENGTH: 1854
   <212> TYPE: PRT
   <213> ORGANISM: Artificial Sequence
<220> FEATURE:
   <223> OTHER INFORMATION: Levivirus and Homo sapiens
<400> SEQUENCE: 64
<210> SEQ ID NO 65
   <211> LENGTH: 5565
   <212> TYPE: DNA
   <213> ORGANISM: Artificial Sequence
<220> FEATURE:
   <223> OTHER INFORMATION:,Levivirus and Homo sapiens
<400> SEQUENCE: 65
<210> SEQ ID NO 66
   <211> LENGTH: 550
   <212> TYPE: PRT
   <213> ORGANISM: Photinus pyralis
<400> SEQUENCE: 66
<210> SEQ ID NO 67
   <211> LENGTH: 1654
   <212> TYPE: DNA
   <213> ORGANISM: Photinus pyralis
<400> SEQUENCE: 67
<210> SEQ ID NO 68
   <211> LENGTH: 805
   <212> TYPE: PRT
   <213> ORGANISM: Artificial Sequence
<220> FEATURE:
   <223> OTHER INFORMATION: Photinus pyralis and Homo sapiens
<400> SEQUENCE: 68
<210> SEQ ID NO 69
   <211> LENGTH: 2418
   <212> TYPE: DNA
   <213> ORGANISM: Artificial Sequence
<220> FEATURE:
   <223> OTHER INFORMATION: Photinus pyralis and Homo sapiens
<400> SEQUENCE: 69
<210> SEQ ID NO 70
   <211> LENGTH: 2274
   <212> TYPE: PRT
   <213> ORGANISM: Artificial Sequence
<220> FEATURE:
   <223> OTHER INFORMATION: Photinus pyralis and Homo sapiens
<400> SEQUENCE: 70
<210> SEQ ID NO 71
   <211> LENGTH: 6825
   <212> TYPE: DNA
   <213> ORGANISM: Artificial Sequence
<220> FEATURE:
   <223> OTHER INFORMATION: Photinus pyralis and Homo sapiens
<400> SEQUENCE: 71
<210> SEQ ID NO 72
   <211> LENGTH: 16
   <212> TYPE: PRT
   <213> ORGANISM: Drosophila melanogaster
<400> SEQUENCE: 72
<210> SEQ ID NO 73
   <211> LENGTH: 51
   <212> TYPE: DNA
   <213> ORGANISM: Drosophila melanogaster
<400> SEQUENCE: 73 cgacagatca agatctggtt tcagaacgca cggatgaagt ggaagaagtg a
   51
<210> SEQ ID NO 74
   <211> LENGTH: 909
   <212> TYPE: PRT
   <213> ORGANISM: Artificial Sequence
<220> FEATURE:
   <223> OTHER INFORMATION: Homo sapiens and Drosophila melanogaster
<400> SEQUENCE: 74
<210> SEQ ID NO 75
   <211> LENGTH: 2730
   <212> TYPE: DNA
   <213> ORGANISM: Artificial Sequence
<220> FEATURE:
   <223> OTHER INFORMATION: Homo sapiens and Drosophila melanogaster
<400> SEQUENCE: 75
<210> SEQ ID NO 76
   <211> LENGTH: 877
   <212> TYPE: PRT
   <213> ORGANISM: Artificial Sequence
<220> FEATURE:
   <223> OTHER INFORMATION: Rattus norvegicus and Drosophila melanogaster
<400> SEQUENCE: 76
<210> SEQ ID NO 77
   <211> LENGTH: 2634
   <212> TYPE: DNA
   <213> ORGANISM: Artificial Sequence
<220> FEATURE:
   <223> OTHER INFORMATION: Rattus norvegicus and Drosophila melanogaster
<400> SEQUENCE: 77
<210> SEQ ID NO 78
   <211> LENGTH: 11
   <212> TYPE: PRT
   <213> ORGANISM: Human immunodeficiency virus type 1
<400> SEQUENCE: 78
<210> SEQ ID NO 79
   <211> LENGTH: 33
   <212> TYPE: DNA
   <213> ORGANISM: Human immunodeficiency virus type 1
<400> SEQUENCE: 79 tacgggcgga agaagcggcg acagaggcga cgg
   33
<210> SEQ ID NO 80
   <211> LENGTH: 904
   <212> TYPE: PRT
   <213> ORGANISM: Artificial Sequence
<220> FEATURE:
   <223> OTHER INFORMATION: Homo sapiens and Human immunodeficiency virus type 1
<400> SEQUENCE: 80
<210> SEQ ID NO 81
   <211> LENGTH: 2715
   <212> TYPE: DNA
   <213> ORGANISM: Artificial Sequence
<220> FEATURE:
   <223> OTHER INFORMATION: Homo sapiens and Human immunodeficiency virus type 1
<400> SEQUENCE: 81
<210> SEQ ID NO 82
   <211> LENGTH: 872
   <212> TYPE: PRT
   <213> ORGANISM: Artificial Sequence
<220> FEATURE:
   <223> OTHER INFORMATION: Rattus norvegicus and Human immunodeficiency virus type 1
<400> SEQUENCE: 82
<210> SEQ ID NO 83
   <211> LENGTH: 2619
   <212> TYPE: DNA
   <213> ORGANISM: Artificial Sequence
<220> FEATURE:
   <223> OTHER INFORMATION: Rattus norvegicus and Human immunodeficiency virus type 1
<400> SEQUENCE: 83
<210> SEQ ID NO 84
   <211> LENGTH: 179
   <212> TYPE: PRT
   <213> ORGANISM: Artificial Sequence
<220> FEATURE:
   <223> OTHER INFORMATION: completely synthesized
<400> SEQUENCE: 84
<210> SEQ ID NO 85
   <211> LENGTH: 747
   <212> TYPE: DNA
   <213> ORGANISM: Artificial Sequence
<220> FEATURE:
   <223> OTHER INFORMATION: completely synthesized
<400> SEQUENCE: 85
<210> SEQ ID NO 86
   <211> LENGTH: 1072
   <212> TYPE: PRT
   <213> ORGANISM: Artificial Sequence
<220> FEATURE:
   <223> OTHER INFORMATION: synthesized and Homo sapiens
<400> SEQUENCE: 86
<210> SEQ ID NO 87
   <211> LENGTH: 3429
   <212> TYPE: DNA
   <213> ORGANISM: Artificial Sequence
<220> FEATURE:
   <223> OTHER INFORMATION: synthesized and Homo sapiens
<400> SEQUENCE: 87
<210> SEQ ID NO 88
   <211> LENGTH: 1040
   <212> TYPE: PRT
   <213> ORGANISM: Artificial Sequence
<220> FEATURE:
   <223> OTHER INFORMATION: synthesized and Rattus norvegicus
<400> SEQUENCE: 88
<210> SEQ ID NO 89
   <211> LENGTH: 3333
   <212> TYPE: DNA
   <213> ORGANISM: Artificial Sequence
<220> FEATURE:
   <223> OTHER INFORMATION: synthesized and Rattus norvegicus
<400> SEQUENCE: 89
<210> SEQ ID NO 90
   <211> LENGTH: 56
   <212> TYPE: PRT
   <213> ORGANISM: Homo sapiens
<400> SEQUENCE: 90
<210> SEQ ID NO 91
   <211> LENGTH: 171
   <212> TYPE: DNA
   <213> ORGANISM: Homo sapiens
<400> SEQUENCE: 91
<210> SEQ ID NO 92
   <211> LENGTH: 949
   <212> TYPE: PRT
   <213> ORGANISM: Artificial Sequence
<220> FEATURE:
   <223> OTHER INFORMATION: Synthesized from two Homo sapiens sequences
<400> SEQUENCE: 92
<210> SEQ ID NO 93
   <211> LENGTH: 2850
   <212> TYPE: DNA
   <213> ORGANISM: Artificial Sequence
<220> FEATURE:
   <223> OTHER INFORMATION: Synthesized from two Homo sapiens sequences
<400> SEQUENCE: 93
<210> SEQ ID NO 94
   <211> LENGTH: 917
   <212> TYPE: PRT
   <213> ORGANISM: Artificial Sequence
<220> FEATURE:
   <223> OTHER INFORMATION: Rattus norvegicus and Homo sapiens
<400> SEQUENCE: 94
<210> SEQ ID NO 95
   <211> LENGTH: 2754
   <212> TYPE: DNA
   <213> ORGANISM: Artificial Sequence
<220> FEATURE:
   <223> OTHER INFORMATION: Rattus norvegicus and Homo sapiens
<400> SEQUENCE: 95
<210> SEQ ID NO 96
   <211> LENGTH: 1005
   <212> TYPE: PRT
   <213> ORGANISM: Artificial Sequence
<220> FEATURE:
   <223> OTHER INFORMATION: Saccaromyces cerevisiae and Homo sapiens and
   Drosophila melanogaster
<400> SEQUENCE: 96
<210> SEQ ID NO 97
   <211> LENGTH: 3018
   <212> TYPE: DNA
   <213> ORGANISM: Artificial Sequence
<220> FEATURE:
   <223> OTHER INFORMATION: Saccaromyces cerevisiae and Homo sapiens and
   Drosophila melanogaster
<400> SEQUENCE: 97
<210> SEQ ID NO 98
   <211> LENGTH: 973
   <212> TYPE: PRT
   <213> ORGANISM: Artificial Sequence
<220> FEATURE:
   <223> OTHER INFORMATION: Saccaromyces cerevisiae and Rattus norvegicus and
   Drosophila melanogaster
<400> SEQUENCE: 98
<210> SEQ ID NO 99
   <211> LENGTH: 2922
   <212> TYPE: DNA
   <213> ORGANISM: Artificial Sequence
<220> FEATURE:
   <223> OTHER INFORMATION: Saccaromyces cerevisiae and Rattus norvegicus and
   Drosophila melanogaster
<400> SEQUENCE: 99
<210> SEQ ID NO 100
   <211> LENGTH: 1238
   <212> TYPE: PRT
   <213> ORGANISM: Artificial Sequence
<220> FEATURE:
   <223> OTHER INFORMATION: synthesized and Saccaromyces cerevisiae and Homo
   sapiens
<400> SEQUENCE: 100
<210> SEQ ID NO 101
   <211> LENGTH: 3717
   <212> TYPE: DNA
   <213> ORGANISM: Artificial Sequence
<220> FEATURE:
   <223> OTHER INFORMATION: synthesized and Saccaromyces cerevisiae and Homo
   sapiens
<400> SEQUENCE: 101
<210> SEQ ID NO 102
   <211> LENGTH: 1206
   <212> TYPE: PRT
   <213> ORGANISM: Artificial Sequence
<220> FEATURE:
   <223> OTHER INFORMATION: synthesized and Saccaromyces cerevisiae and Rattus
   norvegicus
<400> SEQUENCE: 102
<210> SEQ ID NO 103
   <211> LENGTH: 3621
   <212> TYPE: DNA
   <213> ORGANISM: Artificial Sequence
<220> FEATURE:
   <223> OTHER INFORMATION: synthesized and Saccaromyces cerevisiae and Rattus
   norvegicus
<400> SEQUENCE: 103
<210> SEQ ID NO 104
   <211> LENGTH: 1045
   <212> TYPE: PRT
   <213> ORGANISM: Artificial Sequence
<220> FEATURE:
   <223> OTHER INFORMATION: Saccaromyces cerevisiae and two Homo sapiens
   sequences
<400> SEQUENCE: 104
<210> SEQ ID NO 105
   <211> LENGTH: 3138
   <212> TYPE: DNA
   <213> ORGANISM: Artificial Sequence
<220> FEATURE:
   <223> OTHER INFORMATION: Saccaromyces cerevisiae and two Homo sapiens
   sequences
<400> SEQUENCE: 105
<210> SEQ ID NO 106
   <211> LENGTH: 1013
   <212> TYPE: PRT
   <213> ORGANISM: Artificial Sequence
<220> FEATURE:
   <223> OTHER INFORMATION: Saccaromyces cerevisiae and Rattus norvegicus and
   Homo sapiens
<400> SEQUENCE: 106
<210> SEQ ID NO 107
   <211> LENGTH: 3042
   <212> TYPE: DNA
   <213> ORGANISM: Artificial Sequence
<220> FEATURE:
   <223> OTHER INFORMATION: Saccaromyces cerevisiae and Rattus norvegicus and
   Homo sapiens
<400> SEQUENCE: 107
<210> SEQ ID NO 108
   <211> LENGTH: 1000
   <212> TYPE: PRT
   <213> ORGANISM: Artificial Sequence
<220> FEATURE:
   <223> OTHER INFORMATION: Saccaromyces cerevisiae and Homo sapiens and Human immunodeficiency virus type 1
<400> SEQUENCE: 108
<210> SEQ ID NO 109
   <211> LENGTH: 3003
   <212> TYPE: DNA
   <213> ORGANISM: Artificial Sequence
<220> FEATURE:
   <223> OTHER INFORMATION: Saccaromyces cerevisiae and Homo sapiens and Human immunodeficiency virus type 1
<400> SEQUENCE: 109
<210> SEQ ID NO 110
   <211> LENGTH: 968
   <212> TYPE: PRT
   <213> ORGANISM: Artificial Sequence
<220> FEATURE:
   <223> OTHER INFORMATION: Saccaromyces cerevisiae and Rattus norvegicus and Human immunodeficiency virus type 1
<400> SEQUENCE: 110
<210> SEQ ID NO 111
   <211> LENGTH: 2907
   <212> TYPE: DNA
   <213> ORGANISM: Artificial Sequence
<220> FEATURE:
   <223> OTHER INFORMATION: Saccaromyces cerevisiae and Rattus norvegicus and Human immunodeficiency virus type 1
<400> SEQUENCE: 111
<210> SEQ ID NO 112
   <211> LENGTH: 1040
   <212> TYPE: PRT
   <213> ORGANISM: Artificial Sequence
<220> FEATURE:
   <223> OTHER INFORMATION: Levivirus and Homo sapiens and Drosophila
   melanogaster
<400> SEQUENCE: 112
<210> SEQ ID NO 113
   <211> LENGTH: 3123
   <212> TYPE: DNA
   <213> ORGANISM: Artificial Sequence
<220> FEATURE:
   <223> OTHER INFORMATION: Levivirus and Homo sapiens and Drosophila
   melanogaster
<400> SEQUENCE: 113
<210> SEQ ID NO 114
   <211> LENGTH: 1008
   <212> TYPE: PRT
   <213> ORGANISM: Artificial Sequence
<220> FEATURE:
   <223> OTHER INFORMATION: Levivirus and Rattus norvegicus and Drosophila melanogaster
<400> SEQUENCE: 114
<210> SEQ ID NO 115
   <211> LENGTH: 3027
   <212> TYPE: DNA
   <213> ORGANISM: Artificial Sequence
<220> FEATURE:
   <223> OTHER INFORMATION: Levivirus and Rattus norvegicus and Drosophila melanogaster
<400> SEQUENCE: 115
<210> SEQ ID NO 116
   <211> LENGTH: 1273
   <212> TYPE: PRT
   <213> ORGANISM: Artificial Sequence
<220> FEATURE:
   <223> OTHER INFORMATION: Levivirus and Homo sapiens and synthesized
<400> SEQUENCE: 116
<210> SEQ ID NO 117
   <211> LENGTH: 3822
   <212> TYPE: DNA
   <213> ORGANISM: Artificial Sequence
<220> FEATURE:
   <223> OTHER INFORMATION: Levivirus and Homo sapiens and synthesized
<400> SEQUENCE: 117
<210> SEQ ID NO 118
   <211> LENGTH: 1241
   <212> TYPE: PRT
   <213> ORGANISM: Artificial Sequence
<220> FEATURE:
   <223> OTHER INFORMATION: Levivirus and Rattus norvegicus and synthesized
<400> SEQUENCE: 118
<210> SEQ ID NO 119
   <211> LENGTH: 3726
   <212> TYPE: DNA
   <213> ORGANISM: Artificial Sequence
<220> FEATURE:
   <223> OTHER INFORMATION: Levivirus and Rattus norvegicus and synthesized
<400> SEQUENCE: 119
<210> SEQ ID NO 120
   <211> LENGTH: 1080
   <212> TYPE: PRT
   <213> ORGANISM: Artificial Sequence
<220> FEATURE:
   <223> OTHER INFORMATION: Levivirus and two Homo sapiens sequences
<400> SEQUENCE: 120
<210> SEQ ID NO 121
   <211> LENGTH: 3243
   <212> TYPE: DNA
   <213> ORGANISM: Artificial Sequence
<220> FEATURE:
   <223> OTHER INFORMATION: Levivirus and two Homo sapiens sequences
<400> SEQUENCE: 121
<210> SEQ ID NO 122
   <211> LENGTH: 1048
   <212> TYPE: PRT
   <213> ORGANISM: Artificial Sequence
<220> FEATURE:
   <223> OTHER INFORMATION: Levivirus and Rattus norvegicus and Homo sapiens
<400> SEQUENCE: 122
<210> SEQ ID NO 123
   <211> LENGTH: 3147
   <212> TYPE: DNA
   <213> ORGANISM: Artificial Sequence
<220> FEATURE:
   <223> OTHER INFORMATION: Levivirus and Rattus norvegicus and Homo sapiens
<400> SEQUENCE: 123
<210> SEQ ID NO 124
   <211> LENGTH: 1035
   <212> TYPE: PRT
   <213> ORGANISM: Artificial Sequence
<220> FEATURE:
   <223> OTHER INFORMATION: Levivirus and Homo sapiens and Human immunodeficiency virus type 1
<400> SEQUENCE: 124
<210> SEQ ID NO 125
   <211> LENGTH: 3108
   <212> TYPE: DNA
   <213> ORGANISM: Artificial Sequence
<220> FEATURE:
   <223> OTHER INFORMATION: Levivirus and Homo sapiens and Human immunodeficiency virus type 1
<400> SEQUENCE: 125
<210> SEQ ID NO 126
   <211> LENGTH: 1003
   <212> TYPE: PRT
   <213> ORGANISM: Artificial Sequence
<220> FEATURE:
   <223> OTHER INFORMATION: Levivirus and Rattus norvegicus and Human immunodeficiency virus type 1
<400> SEQUENCE: 126
<210> SEQ ID NO 127
   <211> LENGTH: 3012
   <212> TYPE: DNA
   <213> ORGANISM: Artificial Sequence
<220> FEATURE:
   <223> OTHER INFORMATION: Levivirus and Rattus norvegicus and Human immunodeficiency virus type 1
<400> SEQUENCE: 127
<210> SEQ ID NO 128
   <211> LENGTH: 926
   <212> TYPE: PRT
   <213> ORGANISM: Artificial Sequence
<220> FEATURE:
   <223> OTHER INFORMATION: synthesized and Homo sapiens and Drosophila
   melanogaster
<400> SEQUENCE: 128
<210> SEQ ID NO 129
   <211> LENGTH: 2781
   <212> TYPE: DNA
   <213> ORGANISM: Artificial Sequence
<220> FEATURE:
   <223> OTHER INFORMATION: synthesized and Homo sapiens and Drosophila
   melanogaster
<400> SEQUENCE: 129
<210> SEQ ID NO 130
   <211> LENGTH: 894
   <212> TYPE: PRT
   <213> ORGANISM: Artificial Sequence
<220> FEATURE:
   <223> OTHER INFORMATION: synthesized and Rattus norvegicus and Drosophila
   melanogaster
<400> SEQUENCE: 130
<210> SEQ ID NO 131
   <211> LENGTH: 2685
   <212> TYPE: DNA
   <213> ORGANISM: Artificial Sequence
<220> FEATURE:
   <223> OTHER INFORMATION: synthesized and Rattus norvegicus and Drosophila
   melanogaster
<400> SEQUENCE: 131
<210> SEQ ID NO 132
   <211> LENGTH: 1159
   <212> TYPE: PRT
   <213> ORGANISM: Artificial Sequence
<220> FEATURE:
   <223> OTHER INFORMATION: synthesized and Homo sapiens
<400> SEQUENCE: 132
<210> SEQ ID NO 133
   <211> LENGTH: 3480
   <212> TYPE: DNA
   <213> ORGANISM: Artificial Sequence
<220> FEATURE:
   <223> OTHER INFORMATION: synthesized and Homo sapiens
<400> SEQUENCE: 133
<210> SEQ ID NO 134
   <211> LENGTH: 1127
   <212> TYPE: PRT
   <213> ORGANISM: Artificial Sequence
<220> FEATURE:
   <223> OTHER INFORMATION: synthesized and Rattus norvegicus
<400> SEQUENCE: 134
<210> SEQ ID NO 135
   <211> LENGTH: 3384
   <212> TYPE: DNA
   <213> ORGANISM: Artificial Sequence
<220> FEATURE:
   <223> OTHER INFORMATION: synthesized and Rattus norvegicus
<400> SEQUENCE: 135
<210> SEQ ID NO 136
   <211> LENGTH: 966
   <212> TYPE: PRT
   <213> ORGANISM: Artificial Sequence
<220> FEATURE:
   <223> OTHER INFORMATION: synthesized and two Homo sapiens sequences
<400> SEQUENCE: 136
<210> SEQ ID NO 137
   <211> LENGTH: 2901
   <212> TYPE: DNA
   <213> ORGANISM: Artificial Sequence
<220> FEATURE:
   <223> OTHER INFORMATION: synthesized and two Homo sapiens sequences
<400> SEQUENCE: 137
<210> SEQ ID NO 138
   <211> LENGTH: 934
   <212> TYPE: PRT
   <213> ORGANISM: Artificial Sequence
<220> FEATURE:
   <223> OTHER INFORMATION: synthesized and Rattus norvegicus and Homo sapiens
<400> SEQUENCE: 138
<210> SEQ ID NO 139
   <211> LENGTH: 2805
   <212> TYPE: DNA
   <213> ORGANISM: Artificial Sequence
<220> FEATURE:
   <223> OTHER INFORMATION: synthesized and Rattus norvegicus and Homo sapiens
<400> SEQUENCE: 139
<210> SEQ ID NO 140
   <211> LENGTH: 921
   <212> TYPE: PRT
   <213> ORGANISM: Artificial Sequence
<220> FEATURE:
   <223> OTHER INFORMATION: synthesized and Homo sapiens and Human immunodeficiency virus type 1
<400> SEQUENCE: 140
<210> SEQ ID NO 141
   <211> LENGTH: 2766
   <212> TYPE: DNA
   <213> ORGANISM: Artificial Sequence
<220> FEATURE:
   <223> OTHER INFORMATION: synthesized and Homo sapiens and Human immunodeficiency virus type 1
<400> SEQUENCE: 141
<210> SEQ ID NO 142
   <211> LENGTH: 889
   <212> TYPE: PRT
   <213> ORGANISM: Artificial Sequence
<220> FEATURE:
   <223> OTHER INFORMATION: synthesized and Rattus norvegicus and Human immunodeficiency virus type 1
<400> SEQUENCE: 142
<210> SEQ ID NO 143
   <211> LENGTH: 2670
   <212> TYPE: DNA
   <213> ORGANISM: Artificial Sequence
<220> FEATURE:
   <223> OTHER INFORMATION: synthesized and Rattus norvegicus and Human immunodeficiency virus type 1
<400> SEQUENCE: 143

## Claims

1. A method of using vault-like particles as carrier molecules to deliver one or more than one substance to an environmental medium, comprising:
a) providing vault-like particles comprising the one or more than one substance, wherein the vault-like particles:
(i) comprise, consist essentially of or consist of a modified MVP;
(ii) comprise a modified VPARP or modified portion of VPARP comprising at least 150 consecutive residues of VPARP; or
(iii) comprise both a modified MVP and a modified VPARP or modified portion of VPARP comprising at least 150 consecutive residues of VPARP; and
b) administering the vault-like particles to the environmental medium,
wherein the one or more than one substance is selected from the group consisting of an enzyme, a pharmaceutical agent, a plasmid, a polynucleotide, a polypeptide, a chemical sensor, a fluorescent sensor, an ionic sensor, a microorganism sensor, an optical sensor or a pH sensor, or a combination thereof.

2. The method of claim 1, where the vault-like particles are generated using recombinant technology.

3. The method of claim 1, where the vault-like particules:
(i) comprise modified MVP comprising an amino acid sequence added to the N-terminus of MVP which results in one or more than one substance binding domain within the vault-like particle;
(ii) comprise modified MVP comprising an amino acid sequence added to the C-terminus of MVP which results in one or more receptor binding domains; and/or
(iii) comprise VPARP or a portion of VPARP comprising at least 150 consecutive residues of VPARP and comprising an amino acid sequence added to the N-terminus or C-terminus of the VPARP or portion of VPARP comprising at least 150 consecutive residues of VPARP.

4. The method of claim 1(a)(ii), wherein the portion of VPARP optionally comprises residues from about residue 1562 to 1724 or residue 1473 to 1724 of human VPARP, SEQ ID NO: 3.

5. The method of claim 1, further comprising TEP1 or modified TEP1.

6. The method of claim 3(i), wherein the one or more than one substance- binding domain is between 1. and 95 substance-binding domains, is 96 substance-binding domains, or is greater than 96 substance-binding domains.

7. The method of claim 3(i) where the one or more than one substance-binding domain within the vault-like particle is:
one or more than one heavy metal binding domain, optionally where the one or more than one heavy metal binding domain binds a heavy metal selected from the group consisting of cadmium, copper, gold and mercury;
one or more than one polynucleotide-binding domain, optionally a non-specific polynucleotide-binding peptide or a specific polynucleotide-binding peptide.

8. The method or vault-like particles of claim 7, where the peptide added to the N-terminal is a cysteine-rich peptide.

## Patentansprüche

1. Verfahren zur Verwendung von gewölbeartigen Partikeln als Trägermoleküle für die Abgabe von einer oder mehr als einer Substanz an ein Umweltmedium, das Folgendes umfasst:
a) Bereitstellen von gewölbeartigen Partikeln, die die eine oder mehr als eine Substanz umfassen, wobei die gewölbeartigen Partikel:
(i) ein modifiziertes MVP umfassen, im Wesentlichen daraus bestehend oder daraus bestehen;
(ii) eine modifizierte VPARP oder einen modifizierten Abschnitt einer VPARP umfassend mindestens 150 aufeinanderfolgende VPARP-Reste, umfassen; oder
(iii) sowohl ein modifiziertes MVP als auch eine modifizierte VPARP oder einen modifizierten VPARP-Abschnitt mit mindestens 150 aufeinanderfolgenden VPARP-Resten umfassen; und
b) Verabreichen der gewölbeartigen Partikel an das Umweltmedium,
wobei die eine oder mehr als eine Substanz aus der Gruppe bestehend aus einem Enzym, einem Pharmazeutikum, einem Plasmid, einem Polynukleotid, einem Polypeptid, einem chemischen Sensor, einem Fluoreszenzsensor, einem Ionensensor, einem Mikroorganismensensor, einem optischen Sensor oder einem pH-Sensor oder einer Kombination davon ausgewählt ist.

2. Verfahren nach Anspruch 1, wobei die gewölbeartigen Partikel unter Einsatz der Rekombinationstechnik erzeugt werden.

3. Verfahren nach Anspruch 1, wobei die gewölbeartigen Partikel:
i) modifiziertes MVP umfassen, das eine Aminosäuresequenz umfasst, die zu dem N-terminalen Ende des MVP hinzugefügt ist, was zu einer oder mehr als einer Substanzbindungsdomäne innerhalb des gewölbeartigen Partikels führt;
(ii) modifiziertes MVP umfassen, das eine Aminosäuresequenz umfasst, die an das C-terminale Ende des MVP hinzugefügt ist, was zu einer oder mehreren Rezeptorbindungsdomänen führt; und/oder
(iii) VPARP oder einen VPARP-Abschnitt mit mindestens 150 aufeinanderfolgenden VPARP-Resten umfassen und eine Aminosäuresequenz umfassen, die an das N-terminale Ende oder das C-terminale Ende der VPARP oder des VPARP-Abschnitts hinzugefügt ist und die mindestens 150 aufeinanderfolgende VPARP-Reste umfasst.

4. Verfahren nach Anspruch 1(a)(ii), wobei der VPARP-Abschnitt gegebenenfalls Reste von ungefähr Rest 1562 bis 1724 oder Rest 1473 bis 1724 der Human-VPARP, SEQ ID NO: 3, umfasst.

5. Verfahren nach Anspruch 1, das weiterhin TEP1 oder modifiziertes TEP1 umfasst.

6. Verfahren nach Anspruch 3(i), wobei die eine oder mehr als eine Substanzbindungsdomäne zwischen 1 und 95 Substanzbindungsdomänen ist, 96 Substanzbindungsdomänen ist oder mehr als 96 Substanzbindungsdomänen ist.

7. Verfahren nach Anspruch 3(i), wobei die eine oder mehr als eine Substanzbindungsdomäne innerhalb des gewölbeartigen Partikels Folgendes ist:
eine oder mehr als eine Schwermetallbindungsdomäne, wobei gegebenenfalls die eine oder mehr als eine Schwermetallbindungsdomäne ein Schwermetall ausgewählt aus der Gruppe bestehend aus Kadmium, Kupfer, Gold und Quecksilber bindet;
eine oder mehr als eine Polynukleotidbindungsdomäne, gegebenenfalls ein unspezifisches Polynukleotidbindungspeptid oder ein spezifisches Polynukleotidbindungspeptid.

8. Verfahren oder gewölbeartige Partikel nach Anspruch 7, wobei es sich bei dem an das N-terminale Ende hinzugefügte Peptid um ein cysteinreiches Peptid handelt.

## Revendications

1. Procédé d'utilisation de particules de type voûte en tant que molécules porteuses pour acheminer au moins une substance vers un milieu ambiant, comprenant .
a) l'obtention de particules de type voûte comprenant l'au moins une substance, les particules de type voûte :
(i) comprenant une MVP modifiée, étant essentiellement constituées de celle-ci ou étant constituées de celle-ci ;
(ii) comprenant une VPARP modifiée ou une partie modifiée de VPARP comprenant au moins 150 résidus consécutifs de VPARP ; ou
(iii) comprenant à la fois une MVP modifiée et une VPARP modifiée ou une partie modifiée de VPARP comprenant au moins 150 résidus consécutifs de VPARP ; et
b) l'administration des particules de type voûte au milieu ambiant,
dans lequel la ou les substances sont choisies dans le groupe constitué par une enzyme, un agent pharmaceutique, un plasmide, un polynucléotide, un polypeptide, une sonde chimique, une sonde fluorescente, une sonde ionique, une sonde de micro-organisme, une sonde optique ou une sonde de pH ou une association de ceux-ci.

2. Procédé selon la revendication 1, les particules de type voûte étant produites à l'aide de la technologie de recombinaison.

3. Procédé selon la revendication 1, les particules de type voûte :
(i) comprenant une MVP modifiée comprenant une séquence d'acides aminés ajoutée à l'extrémité N de la MVP ce qui a pour résultat au moins un domaine de liaison à des substances au sein de la particule de type voûte ;
(ii) comprenant une MVP modifiée comprenant une séquence d'acides aminés ajoutée à l'extrémité C de la MVP ce qui a pour résultat un ou plusieurs domaines de liaison à des récepteurs ; et/ou
(iii) comprenant une VPARP ou une partie de VPARP comprenant au moins 150 résidus consécutifs de VPARP et comprenant une séquence d'acides aminés ajoutée à l'extrémité N ou à l'extrémité C de la VPARP ou de la partie de VPARP comprenant au moins 150 résidus consécutifs de VPARP.

4. Procédé selon la revendication 1(a)(ii), dans lequel la partie de VPARP comprend facultativement des résidus allant environ du résidu 1562 à 1724 ou du résidu 1473 à 1724 de VPARP humaine, SEQ ID n° : 3.

5. Procédé selon la revendication 1, comprenant en outre une TEP1 ou TEP1 modifiée.

6. Procédé selon la revendication 3(i), dans lequel l'au moins un domaine de liaison à des substances est entre 1 et 95 domaines de liaison à des substances, est 96 domaines de liaison à des substances ou est plus de 96 domaines de liaison à des substances.

7. Procédé selon la revendication 3(i), l'au moins un domaine de liaison à des substances au sein de la particule de type voûte étant :
au moins un domaine de liaison à des métaux lourds, facultativement l'au moins un domaine de liaison à des métaux lourds se liant à un métal lourd choisi dans le groupe constitué par le cadmium, le cuivre, l'or et le mercure ;
au moins un domaine de liaison à des polynucléotides, facultativement un peptide de liaison à des polynucléotides non spécifique ou un peptide de liaison à des polynucléotides spécifique.

8. Procédé ou particules de type voûte selon la revendication 7, le peptide ajouté à l'extrémité N étant un peptide riche en cystéine.
